# EUROPEAN PATENT APPLICATION

(11) **EP 4 497 822 A1**
(43) Date of publication of application: **29.01.2025**
(21) Application number: 23774923.9
(22) Date of filing: 22.03.2023
(51) Int. Cl.: C12N 5/0783, A61K 35/17, A61P 37/06, C12N 5/074, C12N 5/10, C12N 15/09, C12N 15/113

(54) **METHOD FOR PRODUCING REGULATORY T CELLS**

(30) Priority: 23.03.2022 JP 2022047437; 26.09.2022 JP 2022152604
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO, Shin, Kyoto-shi, Kyoto 606-8501 (JP); YANO, Hisashi, Kyoto-shi, Kyoto 606-8501 (JP); SATO, Takayuki, Fujisawa-shi, Kanagawa 251-0012 (JP); SEKIYA, Keiko, Fujisawa-shi, Kanagawa 251-0012 (JP)
(74) Representative: Jones, Nicholas Andrew
(86) International application number: PCT/JP2023/011134
(87) International publication number: WO 2023/182328

(57) **Abstract**

Disclosed are: a method for producing a cell population containing regulatory T cells, the method comprising (1) culturing a cell population containing pluripotent stem cell-derived CD4⁺ T cells in the presence of at least one substance selected from the group consisting of a CDK8 and/or CDK19 inhibitor, a TNFR2 agonist, an mTOR inhibitor, and a TGF-βR agonist; a cell population containing regulatory T cells obtained by the method; and a medicine containing the cell population containing regulatory T cells.

## Description

### Technical Field

The present invention relates to a method for producing a cell population containing regulatory T cells, a cell population containing regulatory T cells obtained by the method, a pharmaceutical containing the cell population containing regulatory T cells, and the like.

### Background of Invention

In recent years, due to the immune response suppression function of regulatory T cells (Treg), research and development of cell therapy using regulatory T cells (Treg therapy) have been promoted worldwide for the purpose of treating various autoimmune diseases. Thus, regulatory T cells are expected to be used in the treatment of graft-versus-host disease (GvHD) and autoimmune diseases, and in the treatment and prevention of inflammatory diseases and allergic diseases.

Regulatory T cells are roughly classified into two types: naturally occurring regulatory T cells (natural Treg: nTreg or thymic Treg: tTreg) and inducible regulatory T cells (induced Treg: iTreg), and it is known that nTreg naturally occurs in the thymus, and iTreg is induced to differentiate from naive T cells in peripheral blood by antigen stimulation and cytokines such as IL-2 and TGF-β. These regulatory T cells are further subdivided according to the type of marker to be expressed in the cell.

When regulatory T cells are removed from a living body, various organ-specific autoimmune diseases develop spontaneously, and at that time, when regulatory T cells are transplanted, the development of autoimmune diseases is prevented, so that regulatory T cells have been considered to play an important role in maintaining peripheral immune self-tolerance. Since then, it has become clear that regulatory T cells can suppress not only autoimmunity but also most immune responses, such as inflammation due to foreign antigens, rejection due to transplantation, infectious immunity, allergies, and tumor immunity. Further, currently, the transcription factor FOXP3 has been revealed as a master regulator of the regulatory T cells.

FOXP3 is a master regulator of Treg, and it is known that FOXP3 is constitutively expressed in Treg, and constitutive expression is not observed in normal T cells (may also be referred to as Tconv (conventional T cells), effector T cells, or inflammatory T cells) having no suppressive function. Therefore, studies on the maintenance of FOXP3 expression in primary Treg and the induction of FOXP3 expression in primary Tconv or bulk CD4 single-positive (SP) T cells have been conducted, and such compounds and cytokines having the activity of maintaining and inducing FOXP3 expression, and combinations thereof have been reported. Such compounds, cytokines, and combinations thereof include AS2863619 as a CDK8/19 inhibitor (NPL 1 and PTL 1), rapamycin as an mTOR inhibitor (NPL 2, NPL 3, NPL 4, and NPL 5), a TNFR2 agonist antibody (NPL 4, NPL 6, and NPL 7), cytokine TGF-β (NPL 1 and NPL 5), and the like.

However, for pluripotent stem cell-derived regulatory T cells, compounds and cytokines maintaining FOXP3 expression, and combinations thereof; compounds and cytokines inducing FOXP3 expression from T cells not expressing FOXP3, and combinations thereof; and the like are not known. It is known that regulatory T cells are difficult to proliferate although an industrial production method with high production efficiency (yield) is desired, and an efficient induction method thereof is expected.

In NPL 8, regarding IPEX (Immune dysregulation, polyendocrinopathy, enteropathy, X-linked) syndrome caused by a mutation in FOXP3, it is described that a gene was transduced into autologous hematopoietic stem cells using a lentiviral vector to restore the FOXP3 expression, and the cells were administered to a model mouse of IPEX syndrome. As a result, it has been reported that hematopoietic stem cells differentiated into functional regulatory T cells and recovered from the autoimmune phenotype. Further, PTL 2 also describes a similar recombinant lentiviral vector containing a nucleotide sequence encoding a human FOXP3 protein.

PTL 3 discloses that it is important to express Mcl-1 together with Foxp3 for improving Treg survival. Further, PTL 4 discloses genetically engineered mammalian cells containing a transgene encoding a lineage commitment factor that promotes differentiation into CD4⁺ Treg. PTL 5 discloses a method for preparing a composition of T cells from stem cells or progenitor cells by culturing three-dimensional cell aggregate including a selected population of stromal cells expressing a Notch ligand and a selected population of stem cells or progenitor cells.

### Citation List

### Patent Literatures

PTL 1: WO2018/139660
PTL 2: WO2019/040655
PTL 3: WO2014/180943
PTL 4: WO2021/092581
PTL 5: WO2017/075389

### Non-patent Literatures

NPL 1: Akamatsu et al., Sci. Immunol. 4, eaaw2707(2019)
NPL 2: Charbonnier et al., Nat. Immunol. 20(9),1208-1219(2019)
NPL 3: MacDonald et al., Clin. Exp. Immunol., 197(1):52-63. (2019)
NPL 4: Urbano et al., Front. Immunol. 9:573(2018)
NPL 5: Schmidt et al, PLoS One., 11(2):e0148474.(2016)
NPL 6: He et al., PLoS One. 11(5), e0156311(2016)
NPL 7: Torrey et al., Sci. Signal. 13, eaba9600(2020)
NPL 8: Cell Stem Cell 24,309-317, February 7,2019

### Summary of Invention

### Technical Problem

An object of the present invention is to provide a method for producing a cell population containing regulatory T cells that can produce regulatory T cells with high efficiency.

### Solution to Problem

The present inventors have conducted intensive studies in order to achieve the above object, and as a result, have obtained a finding that a cell population containing regulatory T cells can be produced with high efficiency by culturing a cell population containing pluripotent stem cell (particularly, iPS cell)-derived CD4⁺ T cells in the presence of at least one substance selected from the group consisting of a CDK8 and/or CDK19 inhibitor, a TNFR2 agonist, an mTOR inhibitor, and a TGF-βR agonist.

The present invention has been completed by further conducting studies based on these findings, and provides the following method for producing a cell population containing regulatory T cells, a cell population containing regulatory T cells, a medicine, and the like.

[1] A method for producing a cell population containing regulatory T cells, the method comprising
   (1) culturing a cell population containing pluripotent stem cell-derived CD4⁺ T cells in the presence of at least one substance selected from the group consisting of a CDK8 and/or CDK19 inhibitor, a TNFR2 agonist, an mTOR inhibitor, and a TGF-βR agonist.
   [1a] A method for producing a cell population containing regulatory T cells, the method comprising
   (1) culturing a cell population containing pluripotent stem cell-derived CD8⁺ T cells in the presence of at least one substance selected from the group consisting of a CDK8 and/or CDK19 inhibitor, a TNFR2 agonist, an mTOR inhibitor, and a TGF-βR agonist.
[2] The method according to [1] or [1a], wherein step (1) is performed in the presence of a CDK8 and/or CDK19 inhibitor and an mTOR inhibitor.
[3] The method according to [1] or [1a], wherein step (1) is performed in the presence of a CDK8 and/or CDK19 inhibitor, an mTOR inhibitor, and a TGF-βR agonist.
[4] The method according to [1] or [1a], wherein step (1) is performed in the presence of a CDK8 and/or CDK19 inhibitor, a TNFR2 agonist, an mTOR inhibitor, and a TGF-βR agonist.
[5] The method according to any one of [1] to [4], wherein the CDK8 and/or CDK19 inhibitor is at least one selected from the group consisting of 4-[1-(2-methyl-1H-benzimidazol-5-yl)-1H-imidazo[4,5-c]pyridin-2-yl]-1,2,5-oxadiazol-3-amine, 3-{1-[1-(4-methoxyphenyl)piperidin-4-yl]-4-methyl-1H-imidazo[4,5-c]pyridin-2-yl}pyrazin-2-amine, and siRNA of CDK8 and/or CDK19.
[6] The method according to any one of [1] to [5], wherein the mTOR inhibitor is rapamycin.
[7] The method according to any one of [1], [1a], and [4] to [6], wherein the TNFR2 agonist is a TNFR2 agonist antibody.
[8] The method according to any one of [1], [1a], and [3] to [7], wherein the TGF-βR agonist is TGF-β.
[9] The method according to any one of [1] to [8], wherein the regulatory T cells are CD25⁺/FOXP3⁺ cells.
[9a] The method according to any one of [1] to [8], wherein the regulatory T cells are CD25⁺/CD127⁻ cells.
[10] The method according to any one of [1] to [9], wherein the CD4⁺ T cells are CD4⁺/CD8⁻ T cells.
[10a] The method according to any one of [1a] to [9a], wherein the CD8⁺ T cells are CD4⁻/CD8⁺ T cells.
[11] The method according to any one of [1] to [10], further comprising (2) inducing differentiation of pluripotent stem cells into a cell population containing CD4⁺ T cells before step (1).
[11a] The method according to any one of [1a] to [9a] and [10a], further comprising (2) inducing differentiation of pluripotent stem cells into a cell population containing CD8⁺ T cells before step (1) .
[11b] The method according to any one of [1] to [11a], comprising (3) introducing a foreign gene (particularly, a gene for expressing a chimeric antigen receptor) into a cell population containing pluripotent stem cells (particularly, iPS cells), CD4⁺ T cells, CD8⁺ T cells, or regulatory T cells.
[12] The method according to any one of [1] to [11b], wherein the pluripotent stem cells are iPS cells.
[13] The method according to [11], [11b], or [12], wherein a three-dimensional cell aggregate is cultured in step (2), the three-dimensional cell aggregate containing cells that can differentiate into the pluripotent stem cell-derived CD4⁺ T cells and stromal cells expressing a Notch ligand.
[13a] The method according to [11a], [11b], or [12], wherein a three-dimensional cell aggregate is cultured in step (2), the three-dimensional cell aggregate containing cells that can differentiate into the pluripotent stem cell-derived CD8⁺ T cells and stromal cells expressing a Notch ligand.
[14] A cell population comprising regulatory T cells obtained by the method according to any one of [1] to [13a].
[15] A medicine comprising the cell population containing regulatory T cells according to [14].
[16] The medicine according to [15], for use in prevention and/or treatment of autoimmune disease, graft-versus-host disease, or transplant rejection.
[17] A method for preventing and/or treating autoimmune disease, graft-versus-host disease, or transplant rejection, the method comprising administering the cell population containing regulatory T cells according to [14] to a subject in need thereof.
[18] The cell population comprising regulatory T cells according to [14], for use in prevention and/or treatment of autoimmune disease, graft-versus-host disease, or transplant rejection.
[19] Use of the cell population comprising regulatory T cells according to [14] in the production of a medicine for use in prevention and/or treatment of autoimmune disease, graft-versus-host disease, or transplant rejection.
[20] The method according to any one of [1] to [13a], wherein the CD4⁺ T cells are cells into which an expression construct is introduced, the expression construct comprising:
   (1) (a) conserved non-coding sequence (CNS) 1, CNS2, and CNS3 of Foxp3 gene;
      (b) a promoter; and
      (c) a nucleic acid encoding FOXP3.
[20a] A method for proliferating a cell population containing regulatory T cells, the method comprising
   (A) culturing a cell population containing regulatory T cells which are primary cultured cells in the presence of a CDK8 and/or CDK19 inhibitor, a TNFR2 agonist, and a TGF-βR agonist.
[20b] The method according to [20a], wherein step (A) is performed in the presence of a CDK8 and/or CDK19 inhibitor, a TNFR2 agonist, and a TGF-βR agonist, and further an mTOR inhibitor.
[20c] The method according to [20a] or [20b], wherein the CDK8 and/or CDK19 inhibitor is at least one selected from the group consisting of 4-[1-(2-methyl-1H-benzimidazol-5-yl)-1H-imidazo[4,5-c]pyridin-2-yl]-1,2,5-oxadiazol-3-amine, 3-{1-[1-(4-methoxyphenyl)piperidin-4-yl]-4-methyl-1H-imidazo[4,5-c]pyridin-2-yl}pyrazin-2-amine, and siRNA of CDK8 and/or CDK19.
[20d] The method according to [20b] or [20c], wherein the mTOR inhibitor is rapamycin.
[20e] The method according to any one of [20a] to [20d], wherein the TNFR2 agonist is a TNFR2 agonist antibody.
[20f] The method according to any one of [20a] to [20e], wherein the TGF-βR agonist is TGF-β.
[20g] The method according to any one of [20a] to [20f], wherein the regulatory T cells are CD25⁺/FOXP3⁺ cells.
[20h] The method according to any one of [20a] to [20f], wherein the regulatory T cells are CD25⁺/CD127⁻ cells.

### Advantageous Effects of Invention

According to the present invention, it is possible to produce regulatory T cells with high efficiency.

### Brief Description of Drawings

Fig. 1 shows flow cytometry (FACS) plots showing cells comprising ATO derived from 4GAD1-4 at 9 week(upper) and from FFI01s04 (bottom) at 6 week.
Fig. 2 shows FACS plots showing the profile of PBMC-derived Tconv and 4GAD1-4-derived CD4SP T cells after initial expansion culture with a base medium.
Fig. 3 shows FACS plots showing bulk (CD4(+) sorted fraction), Tconv (CD4(+) CD25(-) sorted fraction), and Treg (CD4(+) CD25(+) CD127(-) sorted fraction) in PBMC from healthy volunteer donors.
Fig. 4 shows the expansion culture results (FACS plots showing FOXP3 expression) of PBMC-derived Treg in the case of a base medium or in the case of adding AMT or AMRT to the base medium.
Fig. 5 shows the expansion culture results (FACS plots showing FOXP3 expression (left) and graph of expansion ratio (right)) of PBMC-derived Treg in the case of a base medium with IL-2 concentration changed to 100 ng/mL or in the case of adding AMT or AMRT to the base medium.
Fig. 6 shows the expansion culture results (FACS plots showing FOXP3 expression rate) of 4GAD1-4-derived CD4SP T cells in the case of a base medium or in the case of adding AR, ART, AMT, or AMRT to the base medium.
Fig. 7 shows FACS plots of PBMC-derived Treg expanded by culture with addition of AMT to a base medium with IL-2 concentration changed to 100 ng/mL (upper row), PBMC-derived Tconv expanded by culture in a base medium (middle row), and PBMC-derived Tconv expanded by culture with addition of ART to a base medium (lower row).
Fig. 8 shows FACS plots of 4GAD1-4-derived CD4SP T cells expanded by culture in a base medium (upper row) and 4GAD1-4-derived CD4SP T cells expanded in culture with addition of AMRT to a base medium (lower row) .
Fig. 9 is a graph showing the ratio of the copy number of a demethylated FOXP3 TSDR region to the copy number of a demethylated GAPDH gene in each cell type. Since the GAPDH gene is demethylated at a substantially constant rate in any cell, it can be interpreted as a value indicating how many percentage (%) of the cell population is cells having a demethylated TSDR region.
Fig. 10 shows a structure of a CAR molecule used in Example 4.
Fig. 11 shows FACS plots showing PBMC-derived Treg expanded by culture with addition of AMT to a base medium and iPS cell-derived CD4SP T cells expanded by culture with addition of AMRT to a base medium, with (upper) and without (lower) CAR gene transduction.
Fig. 12 shows plots showing the degree of proliferation of cytotoxic T cells (CTL) when CTL, that sorted from the same PBMC as those used in the in vivo experiments described later and expanded by culture, is cocultured with iPS cell-derived CD4SP T cells expanded by culture with IL-2 alone, iPS cell-derived CD4SP T cells expanded by culture with AMRT, or PBMC-derived Treg expanded by culture with AMT (with (lower) or without (upper) CAR gene transduction (lower row)). The further to the right the peak is detected, the more suppressed cell proliferation is.

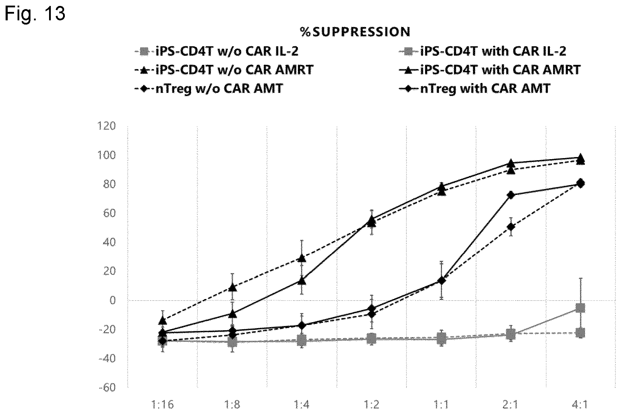

Fig. 13 is a graph showing the proliferation suppression rate (%Suppression) of CTL when one of six types of cells at various ratios: iPS cell-derived CD4SP T cells expanded by culture with IL-2 alone without CAR gene transduction (iPS-CD4T w/o CAR IL-2), iPS cell-derived CD4SP T cells expanded by culture with IL-2 alone with CAR gene transduction (iPS-CD4T with CAR IL-2), iPS cell-derived CD4SP T cells expanded by culture with AMRT without CAR gene transduction (iPS-CD4T w/o CAR AMRT), iPS cell-derived CD4SP T cells expanded by culture with AMRT with CAR gene transduction (iPS-CD4T with CAR AMRT), and PBMC-derived Treg cells expanded by culture with AMT without CAR gene transduction (nTreg w/o CAR AMT), or PBMC-derived Treg cells expanded by culture with AMT with CAR gene transduction (nTreg CAR with CAR AMT)) is co-cultured with CTL. Data values are means ± SEM. n = 3

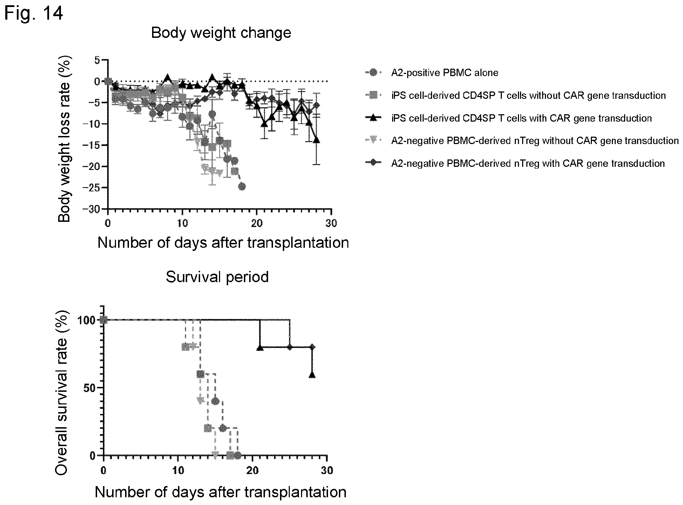

Fig. 14 is a graph showing the body weight (upper) and the overall survival rate (bottom) after transplantation of each group of mice transplanted with one of four types of cells: iPS cell-derived CD4SP T cells expanded by culture with AMRT without CAR gene transduction, iPS cell-derived CD4SP T cells expanded by culture with AMRT with CAR gene transduction, PBMC-derived Treg cells expanded by culture with AMT without CAR gene transduction, or PBMC-derived Treg cells expanded by culture with AMT with CAR gene transduction, and HLA-A2-positive PBMC. Data values are means ± SEM. n = 5 (n = 4 in only iPS cell-derived CD4SP T cells without CAR gene transduction)

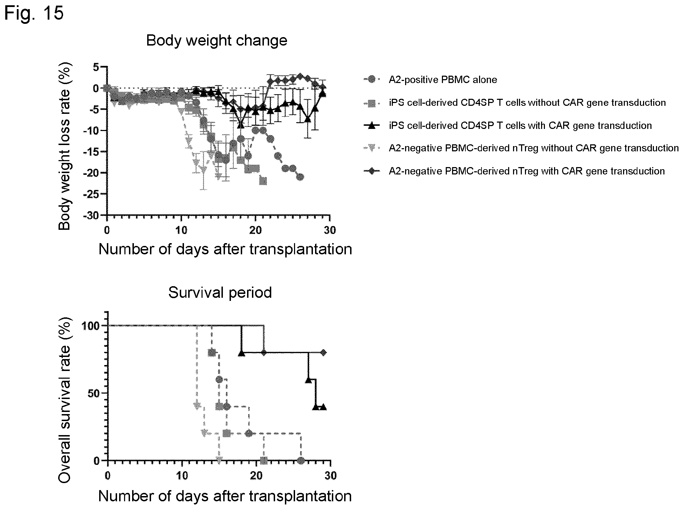

Fig. 15 is a graph showing the body weight (upper) and the overall survival rate (bottom) after transplantation of each group of mice transplanted with one of four types of cells: iPS cell-derived CD4SP T cells expanded by culture with AMRT without CAR gene transduction, iPS cell-derived CD4SP T cells expanded by culture with AMRT with CAR gene transduction, PBMC-derived Treg cells expanded by culture with AMT without CAR gene transduction, PBMC-derived Treg cells expanded by culture with AMT with CAR gene transduction, and HLA-A2-positive PBMC (that is derived from a different HLA-A2-positive PBMC donor than that in Fig. 14). Data values are means ± SEM. n=5
Fig. 16 shows the graph showing the expansion ratio of CNS-Foxp3-transduced iPS cell-derived Treg cultured with a medium with or without AMRT.
Fig. 17 shows the FACS plots of CNS-Foxp3-transduced iPS cell-derived Treg cultured with a medium with or without AMRT.
Fig. 18 shows plots showing the degree of proliferation of PBMC when PBMC derived from a donor is cocultured with CNS-Foxp3-transduced iPS cell-derived Treg expanded by culture with or without addition of AMRT to a medium. The further to the right the peak is detected, the more suppressed cell proliferation is.
Fig. 19A shows the results of pseudotime analysis on integrated single cell RNA-seq datasets of cells in organoid culture and thymocytes (trajectory analysis of single cell RNA-seq data of 3D organoid culture integrated with that of human thymocytes).
Fig. 19B shows the results of pseudotime analysis on integrated single cell RNA-seq datasets of cells in organoid culture and thymocytes (extracted T cell lineage differentiated cells).
Fig. 19C shows the results of pseudotime analysis on integrated single cell RNA-seq datasets of cells in organoid culture and thymocytes (heat map of DEG module based on pseudotime analysis).
Fig. 19D shows the results of pseudotime analysis on integrated single cell RNA-seq datasets of cells in organoid culture and thymocytes (significant top 10 genes of each module cluster).
Fig. 20A shows transcriptome profiles of differentiated cells in 2D and 3D cultures by single-cell RNA sequencing analysis (integrated UMAPs of 3D organoid, 2D culture, and human thymocytes).
Fig. 20B shows transcriptome profiles of differentiated cells in 2D and 3D cultures by single-cell RNA sequencing analysis (feature plots showing CD4, CD8A, and CD8B).
Fig. 20C shows transcriptome profiles of differentiated cells in 2D and 3D cultures by single-cell RNA sequencing analysis (CD4 feature plots of 3D organoid culture samples, thymocytes, and 2D mature cells).
Fig. 21A is a view showing feature plots of designated gene sets for 3D culture, thymocytes, and 2D culture.
Fig. 21B is a view showing feature plots of designated gene sets for 3D culture, thymocytes, and 2D culture.
Fig. 21C is a view showing feature plots of designated gene sets for 3D culture, thymocytes, and 2D culture.

### Description of Embodiments

Hereinafter, embodiments of the present invention will be described in detail.

The term "comprise(s)" or "comprising" means inclusion of the element(s) following the word without limitations thereto. Accordingly, this suggests inclusion of the element(s) following the word, but does not suggest exclusion of any other element. The phrase "consist(s) of" of "consisting of" means inclusion of all the element(s) following the phrase and limitation thereto. Accordingly, the phrase "consist(s) of" or "consisting of" indicates that the enumerated element(s) is required or essential and substantially no other elements exist. The phrase "consist(s) essentially of" or "consisting essentially of" means inclusion of any element following the phrase and limitation of other elements to those that do not affect the activity or effect of the enumerated element(s) specified in the present disclosure. Accordingly, the phrase "consist(s) essentially of" or "consisting essentially of" indicates that the enumerated element(s) is required or essential, but other elements are optional and may exist or not exist depending on whether they affect the activity or effect of the enumerated element(s).

In the present specification, the term "culture" refers to maintenance or/and proliferation of cells in an in-vitro environment. The term "culturing" refers to maintaining or/and proliferating cells outside tissue or outside the body, for example, in a cell culture dish or flask.

In the present specification, the phrase "culturing a cell population in the presence of a substance" refers to, for example, culturing a cell population in a medium containing the substance. Examples of such culture include culture in a medium containing the substance alone or in a medium containing the substance, other differentiation-inducing factors, and the like. When the substance is added to the medium, it may be added directly to the medium, or may be dissolved in an appropriate solvent before use and then added to the medium. Further, culture can also be performed while the substance is immobilized on a substrate or carrier surface during culture.

In the present specification, the term "positive (+)" means that a protein or gene is expressed in detectable amounts by methods known in the art. In the case of a protein that is expressed intracellularly and does not appear on the cell surface (e.g., a transcription factor or a subunit thereof), a reporter protein is expressed together with the protein, and the reporter protein is detected, whereby the target protein can be detected. Gene detection can be performed by using nucleic acid amplification and/or nucleic acid detection methods, such as RT-PCR, microarray, biochip, and RNAseq.

In the present specification, the term "negative (-)" means that the expression level of a protein or gene is less than the lower limit of detection by all or any of the known methods described above, or the degree of expression thereof is low. The lower limit of detection of protein or gene expression may vary depending on the method. The degree of protein or gene expression (low expression or high expression) can be determined by comparison with the result of control cells measured under the same conditions. Examples of the control cells include PBMC-derived Treg and iPS cell-derived CD4SP T cells described in the section of Examples. For example, the degree of CD25 expression in a certain cell population can be determined by comparing the expression level of CD25 in the cell population with the expression level of CD25 in PBMC-derived Tconv (control: known to be low in CD25 expression) using flow cytometry, and when expression equivalent to that of the control is observed, it can be determined as low expression, and when expression is higher than that of the control cells, it can be determined as high expression.

In the present specification, the term "marker" refers to a protein or its gene that is specifically expressed on the cell surface, in the cytoplasm, or in the nucleus of a given cell type. The marker is preferably a "cell surface marker". The "cell surface marker" refers to a protein expressed on the cell surface that can be labeled (stained) with fluorescent substances and that facilitates the detection, condensation, isolation, or the like of cells expressing the cell surface marker. The cell surface marker refers to a gene that is expressed (positive marker) or not expressed (negative marker) specifically in a given cell type, and specifically a substance that is produced (positive marker) or not produced (negative marker) as mRNA by transcription of the gene in the genome or as a protein by translation of the mRNA.

Such cell surface markers can be detected by immunological assays using antibodies specific for the cell surface markers, such as ELISA, immunostaining, and flow cytometry.

In the present specification, the term "expression" is defined as the transcription and/or translation of a specific nucleotide sequence driven by an intracellular promoter.

In the present specification, "pluripotent stem cells" refer to embryonic stem cells (ES cells) and cells with similar pluripotency, i.e. cells with the potential to differentiate into various tissues (endoderm, mesoderm, and ectoderm) of a living body. Examples of cells with the same pluripotency as that of ES cells include "induced pluripotent stem cells" (also referred to as "iPS cells" in the present specification).

In the present specification, "hematopoietic stem cells (HSC)" are multipotent stem cells that can differentiate into blood cells. Hematopoietic stem cells are mainly present in the bone marrow in a human living body, and differentiate into white blood cells (neutrophils, eosinophils, basophils, lymphocytes, monocytes, and macrophages), red blood cells, platelets, mast cells, dendritic cells, and the like. In the present specification, hematopoietic stem cells (HSC) can be positive to CD34 and negative to CD7 (CD34⁺/CD7⁻) . In the present specification, "/" used in the phrase "CD34⁺/CD7⁻" etc. means "and".

In the present specification, "hematopoietic progenitor cells" (HPC) are cells that have the ability to differentiate into blood cells but do not have the ability to self-renew as much as stem cells. Hematopoietic progenitor cells are mainly present in the bone marrow in a human living body. In the present specification, hematopoietic progenitor cells (HPC) can be positive to CD34 and negative to CD43 (CD34⁺/CD43⁺) . Further, HPC can be positive to CD24, CD62L, CD90, CD143, CD263, Notch3, CD32, CD39, CD49a, CD164, CD317, CD200, CD218a, CD7, CD144, CD56, CD226, CD262, and CD325 and negative to CD49f, CD51, CD102, CD42b, CD61, CD62P, CD69, CD102, and CD156c, as described in WO2018/199186.

In the present specification, "hemogenic endothelial cells (HEC)" are cells that express CD34 but do not express CD43, CD184, and CD73 (CD34⁺/CD43⁻/CD184⁻/CD73⁻) (CD34⁺/CD43⁻/CD184⁻/CD73⁻ cells do not express CD7, and CD34⁺/CD43⁻/CD184⁻/CD73⁻ cells are thus also referred to as "CD34⁺/CD7⁻/CD43⁻/CD184⁻/CD73⁻ cells") .

In the present specification, "progenitor T cells (proT)" refer to hematopoietic cells produced in the process of differentiation from hematopoietic stem cells into CD3-positive T cells in a human living body, and are positive to CD34 and CD7 (CD34⁺/CD7⁺ cells) . Further, in the present specification, proT can be negative to CD43, CD1a and/or CD116.

In the present specification, "mesodermal progenitor cells" refer to, for example, cells that express at least one marker gene selected from the group consisting of T (synonymous with Brachyury), KDR, FOXF1, FLK1, BMP4, MOX1, and SDF1. Mesodermal progenitor cells are not distinguished from mesodermal cells, and cells with weak expression of the above marker genes may be referred to as "mesodermal progenitor cells".

In the present specification, "regulatory T cells" refer to T cells that have the ability to inhibit the activation of effector T cells when stimulated via T cell receptors and that are responsible for the suppression of immune responses (immune tolerance). Regulatory T cells are generally CD25⁺/FOXP3⁺ cells or CD25⁺/CD127⁻ cells, and CD4⁺ or CD8⁺ cells are present therein. In the present invention, the regulatory T cells may be CD4⁺ cells (i.e., CD4⁺/CD25⁺/FOXP3⁺ or CD4⁺/CD25⁺/CD127⁻) or CD8⁺ cells (i.e., CD8⁺/CD25⁺/FOXP3⁺ or CD8⁺/CD25⁺/CD127⁻), and more preferably CD4⁺ cells. Further, the transcription factor FOXP3 is known as a master regulator of CD4⁺/CD25⁺ regulatory T cells.

In the present specification, "CD4⁺ T cells" refer to, among T cells, those with surface antigens CD4 that are positive. Preferably, CD4⁺ T cells are negative to CD8 (CD4⁺/CD8⁻, CD4 single positive (SP)), and in this case, since T cells can be identified by surface antigens CD3 and CD45 being positive, CD4⁺ T cells can be identified as cells that are negative to CD8 and positive to CD4, CD3, and CD45 (CD4⁺/CD8⁻/CD3⁺/CD45⁺ cells) . CD4⁺ T cells are cells that are negative to CD25 and FOXP3 and positive to CD127. Examples of the CD4⁺ T cells include helper T cells.

In the present specification, "CD8⁺ T cells" refer to, among T cells, those with surface antigens CD8 that are positive. Preferably, CD8⁺ T cells are negative to CD4 (CD4⁻/CD8⁺, CD8 single positive (SP)), and in this case, since T cells can be identified by surface antigens CD3 and CD45 being positive, CD8⁺ T cells can be identified as cells that are negative to CD4 and positive to CD8, CD3, and CD45 (CD4⁻/CD8⁺/CD3⁺/CD45⁺ cells) . CD8⁺ T cells are cells that are negative to CD25 and FOXP3 and positive to CD127. Examples of the CD8⁺ T cells include cytotoxic T cells.

In the present specification, "stromal cells" are cells constituting the connective tissue that supports parenchymal cells in a living tissue, and particularly refer to stromal cells capable of producing an ATO that produces T cells, typically, stromal cells contained in hematopoietic tissues such as bone marrow or thymus, and examples thereof include fibroblasts, blood cells, mesenchymal stem cells, endothelial cells, smooth muscle cells, epithelial cells, and tissue stem cells. The stromal cells may be established cells such as MS-5, OP9, and S17, which are mouse bone marrow cell strains, or HS-5 and HS-27a, which are human stromal cell strains, or may be primary cells collected from humans or the like, and cells differentiated from pluripotent stem cells (iPS cells, etc.), and in the case of cells induced to differentiate from pluripotent stem cells of humans or the like, stromal cells induced to differentiate from human iPS cells (particularly, fibroblasts induced to differentiate from human iPS cells) are preferred.

In the present specification, "CD4CD8 double-positive T cells" refer to, among T cells, those with surface antigens CD4 and CD8 that are both positive (CD8⁺/CD4⁺), and since T cells can be identified by surface antigens CD3 and CD45 being positive, CD4CD8 double-positive T cells can be identified as cells that are positive to CD4, CD8, CD3, and CD45 (CD8⁺/CD4⁺/CD3⁺/CD45⁺ cells) .

In the present specification, "CD4CD8 double-negative T cells" refer to, among T cells, those with surface antigens CD4 and CD8 that are both negative (CD8⁻/CD4⁻), and since T cells can be identified by surface antigens CD3 and CD45 being positive, CD4CD8 double-negative T cells can be identified as cells that are negative to CD4 and CD8 and positive to CD3 and CD45 (CD8⁻/CD4⁻/CD3⁺/CD45⁺ cells) .

In the present specification, "cell population" means two or more cells of the same type or different types. The "cell population" also means a mass of cells of the same type or different types.

The various cells used in the present invention are preferably cells certified by the Good Manufacturing Practice (GMP), from the viewpoint of therapeutic applications.

Examples of pluripotent stem cells include embryonic stem cells (ES cells), induced pluripotent stem cells (iPS cells), embryonal carcinoma cells (EC cells), embryonic germ stem cells (EG cells), and Muse cells; and preferably iPS cells (more preferably human iPS cells). When the pluripotent stem cells are ES cells or any cells derived from the human embryo, the cells may be produced by destroying the embryo or without destroying the embryo, and preferably cells produced without destroying the embryo.

Regarding "ES cells", various mouse ES cell strains established by Ingenious Targeting Laboratory, RIKEN, etc. can be used as mouse ES cells, and various human ES cell strains established by the University of Wisconsin, NIH, RIKEN, Kyoto University, National Center for Child Health and Development, Cellartis, etc. can be used as human ES cells. Usable examples of human ES cell strains include CHB-1 to CHB-12 strains, RUES1 strain, RUES2 strain, HUES1 to HUES28 strains, etc. furnished by ESI Bio; H1 strain, H9 strain, etc. furnished by WiCell Research; and KhES-1 strain, KhES-2 strain, KhES-3 strain, KhES-4 strain, KhES-5 strain, SSES1 strain, SSES2 strain, SSES3 strain, etc. furnished by RIKEN.

"Induced pluripotent stem cells" refer to cells obtained by reprogramming mammalian somatic cells or undifferentiated stem cells by introduction with specific factors (nuclear reprogramming factors). Currently, there are many different types of induced pluripotent stem cells. Usable examples include iPS cells established by Yamanaka et al. by introducing four factors, Oct3/4, Sox2, Klf4, and c-Myc, into mouse fibroblasts (Takahashi K, Yamanaka S., Cell, (2006) 126: 663-676), as well as human cell-derived iPS cells established by introducing the same four factors into human fibroblasts (Takahashi K, Yamanaka S., et al. Cell, (2007) 131: 861-872.), Nanog-iPS cells established by introducing the above four factors, followed by screening using the expression of Nanog as an index (Okita, K., Ichisaka, T., and Yamanaka, S. (2007). Nature 448, 313-317.), iPS cells produced by a method that does not include c-Myc (Nakagawa M, Yamanaka S., et al. Nature Biotechnology, (2008) 26, 101-106), and iPS cells established by introducing six factors by a virus-free method (Okita K et al. Nat. Methods 2011 May; 8(5): 409-12, Okita K et al. Stem Cells. 31(3): 458-66.). Other usable examples include induced pluripotent stem cells produced by Thomson et al. and established by introducing four factors, OCT3/4, SOX2, NANOG, and LIN28 (Yu J., Thomson JA. et al., Science (2007) 318: 1917-1920.), induced pluripotent stem cells produced by Daley et al. (Park IH, Daley GQ. et al., Nature (2007) 451: 141-146), induced pluripotent stem cells produced by Sakurada et al. (JP2008-307007A), and the like.

Other usable examples are any of the induced pluripotent stem cells known in the art and described in all of the published articles (e.g., Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No 7, 795-797), or patents (e.g., JP2008-307007A, JP2008-283972A, US2008/2336610, US2009/047263, WO2007/069666, WO2008/118220, WO2008/124133, WO2008/151058, WO2009/006930, WO2009/006997, and WO2009/007852) .

As induced pluripotent stem cell strains, various iPS cell strains established by NIH, RIKEN, Kyoto University, etc. can be used. Examples of human iPS cell strains include HiPS-RIKEN-1A strain, HiPS-RIKEN-2A strain, HiPS-RIKEN-12A strain, and Nips-B2 strain (RIKEN); Ff-I01s04 strain, QHJI strain, RWMH strain, DRXT strain, RJWI strain, YZWJ strain, ILCL strain, GLKV strain, 253G1 strain, 201B7 strain, 409B2 strain, 454E2 strain, 606A1 strain, 610B1 strain, and 648A1 strain (Kyoto University); and the like.

The "nucleic acid" may be any molecule obtained by polymerizing a nucleotide and a molecule having the same function as the nucleotide, examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog, and the nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid, and specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

The method for producing a cell population containing regulatory T cells of the present invention (also referred to simply as "the production method of the present invention" in the present specification) characteristically comprises the following step:
1) culturing a cell population containing pluripotent stem cell-derived CD4⁺ T cells in the presence of at least one substance selected from the group consisting of a CDK8 and/or CDK19 inhibitor, a TNFR2 agonist, an mTOR inhibitor, and a TGF-βR agonist.

The CDK8 (cyclin-dependent kinase 8) and/or CDK19 inhibitor is defined as a substance that inhibits the function of CDK8 and/or CDK19, particularly a substance that inhibits the kinase activity of CDK8 and/or CDK19, and may be an inhibitor of both CDK8 and CDK19 or may be an inhibitor of any one of them. Examples of the CDK8 and/or CDK19 inhibitor include 4-[1-(2-methyl-1H-benzimidazol-5-yl)-1H-imidazo[4,5-c]pyridin-2-yl]-1,2,5-oxadiazol-3-amine (AS2863619) (hereinafter also referred to as "compound 1"); 3-{1-[1-(4-methoxyphenyl)piperidin-4-yl]-4-methyl-1H-imidazo[4,5-c]pyridin-2-yl}pyrazin-2-amine (AS3334366) (hereinafter also referred to as "compound 2"); siRNA, shRNA, dsRNA, miRNA, and antisense nucleic acid for genes encoding CDK8 and/or CDK19 and/or transcripts thereof, and expression vectors expressing them; compounds described in US8598344, WO2013/116786, Proc. Natl. Acad. Sci. U.S.A. 109 13799-13804 (2012), WO2013/001310, WO2013/040153, WO2014/029726, WO2014063778, WO2014/072435, WO2014/090692, WO2014/106606, WO2014/123900, WO2014/154723, WO2014/194245, WO2015/049325, WO2015/100420, WO2015/144290, WO2015/159937, and WO2015/159938 (particularly, (2E)-N-(2-fluoro-4-(morpholine-4-yl methyl)phenyl)-3-(4-(1-methyl-1H-pyrazol-4-yl)pyridin-3-yl)acrylamide (hereinafter also referred to as "compound 3") and (2E)-3-(4-(1-cyclopropyl-1H-pyrazol-4-yl)pyridin-3-yl)-N-(1-methyl-1H-indol-5-yl)acrylamide (hereinafter also referred to as "compound 4")); compounds described in WO2016/009076 and WO2018/159805 (particularly, 8-(2,4-difluorophenoxy)-1-methyl-4,5-dihydro-1H-thieno[3,4-g]indazol-6-carboxamide (hereinafter also referred to as "compound 5"), 4-((4-fluorophenyl)sulfonyl)-3-(2-(imidazo[1,2-b]pyridazin-6-yl sulfanyl)ethyl)-3,4-dihydroquinoxalin-2(1H)-one (hereinafter also referred to as "compound 6"), 2-(benzylamino)-4-(1H-pyrrolo[2,3-b]pyridin-3-yl)benzamide (hereinafter also referred to as "compound 7"), 3-(3-(benzyloxy)phenyl)-1H-pyrrolo[2,3-b]pyridin (hereinafter also referred to as "compound 8"), 4-(4-(2,3-dihydro-1,4-benzodioxin-6-yl)-1H-pyrazol-3-yl)benzen-1,3-diol (particularly, trifluoroacetic acid salt thereof) (hereinafter also referred to as "compound 9"), N-butyl-8-(4-methoxyphenyl)-1,6-naphthyridin-2-carboxamide, and 8-(4-methylphenyl)-N,N-dipropyl-1,6-naphthyridin-2-carboxamide (particularly, trifluoroacetic acid salt thereof) (hereinafter also referred to as "compound 10")); compounds described in WO2022/107877 (particularly, diethyl (E)-(4-(3-(5-(4-fluorophenyl)-1-methyl-1H-pyrazol-4-yl)acrylamide)benzyl)phosphonate (hereinafter also referred to as "compound 11"), 2-(4-(4-(isoquinolin-4-yl)phenyl)-1H-pyrazol-1-yl)-N,N-dimethylacetamide (BI-1347), and 4-((2-(6-(4-methylpiperazin-1-carbonyl)naphthalen-2-yl)ethyl)amino)quinazolin-6-carbonitrile (Senexin B)); MSC2530818 (CAS No.: 1883423-59-3) and CCT251921 (CAS No.: 1607837-31-9), and the like. Preferred among these are compound 1, compound 2, compound 3, compound 4, compound 5, compound 6, compound 7, compound 8, compound 9, compound 10, compound 11, BI-1347, Senexin B, MSC2530818, CCT251921, and siRNA for genes encoding CDK8 and/or CDK19 and/or transcripts thereof; more preferred are compound 1, compound 2, and siRNA for genes encoding CDK8 and/or CDK19 and/or transcripts thereof; and further preferred is compound 1. The CDK8 and/or CDK19 inhibitor can be used singly or in combination of two or more kinds thereof. In addition, as a method for inhibiting CDK8 and/or CDK19, it can also be performed by knocking out a gene encoding CDK8 and/or CDK19. As a method for knocking out such a gene, for example, a method known in the art such as genome editing using ZFN, TALEN, CRISPR/Cas system, or the like can be used.

The TNFR2 (tumor necrosis factor receptor-2) agonist is defined as a substance that can bind to TNFR2 to activate TNFR2 signaling, and examples thereof include antibodies (e.g., anti-TNFR2 antibody), peptides, low-molecular-weight compounds, proteins, and the like. Examples of the TNFR2 agonist antibody include monoclonal antibodies binding to TNFR2, such as clone MR2-1 (Hycult Biotech) and clone MAB2261 (R&D Systems). The TNFR2 agonist may be TNF-α mutein that binds to only TNFR2 as an agonist. The TNFR2 agonist can be used singly or in combination of two or more kinds thereof.

The TNFR2 agonist is preferably a TNFR2 agonist antibody. The antibody may be a functional fragment thereof, and examples of the functional fragment include Fd, Fv, Fab, F(ab'), F(ab)₂, F(ab')₂, single-chain Fv (scFv), diabody, triabody, tetrabody, and minibody. Examples of the antibody include those derived from animals, such as mice, rats, cows, rabbits, goats, sheep, and guinea pigs. The isotype of the antibody is not particularly limited, and examples thereof include IgG (IgG1, IgG2, IgG3, and IgG4), IgA, IgD, IgE, and IgM. The antibody may be a monoclonal antibody or a polyclonal antibody, and preferably a monoclonal antibody, and the antibody may be a humanized antibody, a chimeric antibody, a multispecific antibody (e.g., a bispecific antibody), or the like. The antibody can be produced by a known method, and for example, the antibody can be produced by constructing an expression vector containing a nucleic acid encoding the antibody, and culturing a transformant into which the nucleic acid is introduced, or culturing a hybridoma that produces the antibody.

The mTOR (mechanistic target of rapamycin) inhibitor is defined as a substance that inhibits the function of mTOR, and these include a substance that inhibits the function of mTOR itself, and a substance that inhibits the function of mTOR complex 1 (mTORC1) and mTOR complex 2 (mTORC2), which are mTOR complexes. Examples of the mTOR inhibitor include rapamycin or derivatives thereof, everolimus, temsirolimus, ridaforolimus, sirolimus, KU0063794, AZD805, AZD8055, WYE-354, WAY-600, WYE-687, Pp121, Pp242, Dactolisib, Sapanisertib, Omipalisib, Vistusertib, Torin 1, Torin 2, and the like. Other examples of the mTOR inhibitor include siRNA, shRNA, dsRNA, miRNA, and antisense nucleic acid for genes encoding mTOR and/or transcripts thereof, and expression vectors expressing them; and further include siRNA, shRNA, dsRNA, miRNA, and antisense nucleic acid for genes encoding enzymes that phosphorylate and activate mTOR and/or transcripts thereof, and expression vectors expressing them. Of these, the mTOR inhibitor is preferably rapamycin or a derivative thereof, and more preferably rapamycin. The mTOR inhibitor can be used singly or in combination of two or more kinds thereof.

The TGF-βR Transforming Growth Factor-β Receptor) agonist is defined as a substance that can bind to TGF-βR to activate TGF-βR signaling. Examples of the TGF-βR agonist include factors belonging to the TGF-β superfamily, and the TGF-β superfamily includes TGF-β family, activin family, and BMP (bone morphogenetic protein) family. Examples of the factors belonging to the TGF-β superfamily include TGF-β (TGF-β1, TGF-β2, and TGF-β3), activin A, activin B, GDF-8, GDF-11, and the like. The TGF-βR agonist is preferably TGF-β. The TGF-βR agonist can be used singly or in combination of two or more kinds thereof.

Further, the CDK8 and/or CDK19 inhibitor, the TNFR2 agonist, the mTOR inhibitor, and the TGF-βR agonist can be used in free form or in salt form. Examples of salts include salts with inorganic bases, such as sodium salts, magnesium salts, potassium salts, calcium salts, and aluminum salts; salts with organic bases, such as methylamine salts, ethylamine salts, and ethanolamine salts; salts with basic amino acids, such as lysine, ornithine, and arginine; and ammonium salts. These salts may be acid addition salts, and specific examples of such salts include addition salts with mineral acids, such as hydrochloric acid, hydrobromic acid, hydroiodic acid, sulfuric acid, nitric acid, and phosphoric acid; organic acids, such as formic acid, acetic acid, propionic acid, oxalic acid, malonic acid, malic acid, tartaric acid, fumaric acid, succinic acid, lactic acid, maleic acid, citric acid, methanesulfonic acid, and ethanesulfonic acid; and acidic amino acids, such as aspartic acid and glutamic acid. The CDK8 and/or CDK19 inhibitor, the TNFR2 agonist, the mTOR inhibitor, and the TGF-βR agonist also include hydrates, solvates, polymorphs, and the like.

When the CDK8 and/or CDK19 inhibitor, the TNFR2 agonist, the mTOR inhibitor, and the TGF-βR agonist contain asymmetric carbon, these include R-isomers, S-isomers, and mixtures containing both isomers in any ratio (e.g., racemates).

siRNA (small interfering RNA) is a double-stranded oligo RNA including an RNA having a sequence complementary to a nucleotide sequence of mRNA of a target gene or a partial sequence thereof (target nucleotide sequence) and a complementary strand thereof. Also included in the siRNA are: RNA (small hairpin RNA (shRNA)) which is a single-stranded RNA in which a sequence complementary to a target nucleotide sequence (first sequence) and a complementary sequence thereof (second sequence) are linked via a hairpin loop portion, wherein the first sequence forms a double strand structure with the second sequence by taking a hairpin loop type structure; and a dumbbell-shaped nucleic acid in which both ends of the double strand structure of the first sequence and the second sequence are closed in a loop structure.

The siRNA may have an overhang at the 5'-terminal or 3'-terminal of one or both of the sense strand and the antisense strand. The overhang is formed by addition of one to several (e.g., 1, 2, or 3) bases at the terminal of a sense strand and/or an antisense strand. The base length of the siRNA is not particularly limited as long as RNA interference can be induced, and for example, one side strand has a 10 to 50 bases, 15 to 30 bases, or 21 to 27 bases.

The siRNA is siRNA targeting mRNA encoding CDK8 and/or CDK19, mTOR, or the like, and can be designed on the basis of information on the base sequence of the mRNA, and the sequence is not particularly limited as long as RNA interference can be induced. The siRNA can be obtained by chemical synthesis using a known method or produced using a genetic recombination technique.

The concentration of the CDK8 and/or CDK19 inhibitor in the medium is not particularly limited as long as the regulatory T cells can be produced, and is suitably adjusted according to the type of CDK8 and/or CDK19 inhibitor used, etc. The concentration of the CDK8 and/or CDK19 inhibitor is, for example, 0.1 to 300 nM, and preferably 0.5 to 150 nM.

The concentration of the TNFR2 agonist in the medium is not particularly limited as long as the regulatory T cells can be produced, and is suitably adjusted according to the type of TNFR2 agonist used, etc. The concentration of the TNFR2 agonist is, for example, 0.0001 to 100 µg/mL, and preferably 0.01 to 10 µg/mL.

The concentration of the mTOR inhibitor in the medium is not particularly limited as long as the regulatory T cells can be produced, and is suitably adjusted according to the type of mTOR inhibitor used, etc. The concentration of the mTOR inhibitor is, for example, 1 to 100 nM, and preferably 10 to 100 nM.

The concentration of the TGF-βR agonist in the medium is not particularly limited as long as the regulatory T cells can be produced, and is suitably adjusted according to the type of TGF-βR agonist used, etc. The concentration of the TGF-βR agonist is, for example, 0.1 to 100 ng/mL, and preferably 1 to 50 ng/mL.

In step (1), culture is performed in the presence of at least one substance selected from the group consisting of a CDK8 and/or CDK19 inhibitor (hereinafter, also abbreviated as "A"), a TNFR2 agonist (hereinafter, also abbreviated as "M"), an mTOR inhibitor (hereinafter, also abbreviated as "R"), and a TGF-βR agonist (hereinafter, also abbreviated as "T"). These four substances are used alone or in combination of AM, AR, AT, MR, MT, RT, AMR, ART, AMT, MRT, or AMRT. These four substances are preferably used in combination of AR, ART, or AMRT, and more preferably used in combination of AMRT.

The medium used in the culture of step (1) uses a medium used for culturing animal cells as the basal medium, and contains at least one substance selected from the group consisting of the CDK8 and/or CDK19 inhibitor, the TNFR2 agonist, the mTOR inhibitor, and the TGF-βR agonist described above. The basal medium is not particularly limited as long as it can be used for culturing of animal cells, and examples thereof include AIM V, X-VIVO-15, NeuroBasal, EGM2, TeSR, BME, BGJb, CMRL 1066, Glasgow's MEM, Improved MEM Zinc Option, IMDM, 199 medium, Eagle's MEM, ∝MEM, DMEM, Ham, RPMI-1640, Fischer's medium, and the like. These media may be used singly or as a mixture of two or more kinds thereof.

The medium may contain serum or may be serum-free. The medium may further contain serum replacements (e.g., albumin, transferrin, Knockout Serum Replacement (KSR), fatty acid, insulin, collagen precursor, minor element, 2-mercaptoethanol, 3'-thiolglycerol, ITS-supplement, B27 (trademark) supplement, etc.). Serum replacements can be used singly or in combination of two or more kinds thereof.

The medium may further contain one or more substances of lipids, amino acids (non-essential amino acids etc.), L-glutamine, vitamins, growth factors, cytokines (IL-2 etc.), beads to which a CD3 antibody and/or a CD28 antibody is bound, antibiotics, antioxidants, pyruvic acid, buffers, inorganic salts, and the like, and particularly preferably contains IL-2. As the medium, it is desirable to use a chemically-defined medium that does not contain materials with unknown components, such as serum, because the difference in medium among lots can be reduced, and cells with stable quality can be prepared.

When the medium contains IL-2, the concentration of IL-2 is, for example, 1 to 500 ng/mL and preferably 1 to 300 ng/mL. In particular, when a cell population containing pluripotent stem cell-derived CD4⁺ T cells is cultured in step (1), the concentration thereof is, for example, preferably 1 to 100 ng/mL, more preferably 1 to 50 ng/mL, further preferably 3 to 30 ng/mL, and particularly preferably 5 to 15 ng/mL. Further, when a cell population containing regulatory T cells as primary cultured cells is expanded by culture using a combination of AMT in step (A) described later, in order to highly maintain the FOXP3 expression rate, the concentration of IL-2 contained in the medium is, for example, set to preferably 30 to 300 ng/mL and more preferably 50 to 200 ng/mL.

The pH of the medium is generally 7.0 to 7.8 and preferably 7.2 to 7.6. In order to prevent contamination before use, the medium is preferably sterilized by a method such as filtration, UV irradiation, heat sterilization, or radiation.

The culture is carried out in the presence or absence of feeder cells. Since the production method of the present invention can stably produce regulatory T cells having uniform properties without mixing substances whose components are not clear, it is desirable to carry out the production method in the absence of feeder cells.

The culture conditions of the production method of the present invention are not particularly limited, and the culture temperature is, for example, about 37 to 42°C, preferably about 37 to 39°C, the CO₂ concentration is, for example, 2% to 10%, preferably 2 to 5%, and the oxygen concentration is, for example, 1 to 20%, preferably 5 to 20%.

The culture period is also not particularly limited, and can be suitably determined by a person skilled in the art while monitoring the number of regulatory T cells, and is, for example, 10 days or more, preferably 1 week or more, and more preferably 2 weeks or more. The upper limit of the culture period is not particularly limited, and is, for example, 35 days or less, preferably 28 days or less, and more preferably 21 days or less. In the culture in the present invention, passage may be performed as many times as necessary to obtain the desired amount of regulatory T cells, or addition and replacement of medium may be performed. The culture in the present invention can be performed using a known CO₂ incubator. The culture container is not particularly limited, and can be suitably selected from plates, dishes, petri dishes, flasks, bags, bottles, tanks (culture tanks), bioreactors, and the like.

Pluripotent stem cells and cells for producing pluripotent stem cells used in the present invention may be derived from humans, or may be derived from mammals other than humans (non-human mammals), and are preferably derived from humans. Examples of the non-human mammals include mice, rats, hamsters, guinea pigs, rabbits, dogs, cats, pigs, cows, horses, sheep, and monkeys. Further, universalized iPS cells (having a specific HLA that does not cause immune rejection in a large number of patients, or having HLA knocked out) can also be used.

The cell population containing CD4⁺ T cells used in the production method of the present invention is a cell population containing CD4⁺ T cells induced to differentiate from pluripotent stem cells (particularly iPS cells (especially human iPS cells)). The proportion (number of cells) of CD4⁺ T cells contained in the cell population containing CD4⁺ T cells is, for example, 5% or more, preferably 50% or more, and more preferably 90% or more, and the upper limit is, for example, 100% or less.

The production method of the present invention may further include separating the regulatory T cells in order to concentrate the obtained regulatory T cells. The separation of the regulatory T cells can be performed by a known method such as a method using flow cytometry or a magnetic cell separation method.

According to the production method of the present invention, a cell population containing regulatory T cells with a high content ratio of regulatory T cells can be produced. The proportion (number of cells) of regulatory T cells contained in the cell population containing regulatory T cells is, for example, 10% or more, preferably 40% or more, and more preferably 80% or more, and the upper limit is, for example, 90% or less and more preferably 100% or less.

According to the production method of the present invention, a cell population containing regulatory T cells with a high content rate of regulatory cells showing a high suppressive function (e.g., Helios-positive cells as functional markers of suppressibility, cells actually showing high suppressibility in vitro and/or in vivo) can be produced. The proportion (number of cells) of regulatory T cells contained in the cell population containing regulatory T cells is, for example, 10% or more, preferably 50% or more, and more preferably 80% or more, and the upper limit is, for example, 90% or less and more preferably 100% or less.

The production method of the present invention can also be applied to a cell population containing CD8⁺ T cells in addition to a cell population containing CD4⁺ T cells.

In another embodiment of the present invention, a method for proliferating a cell population containing regulatory T cells (also referred to simply as "the proliferation method of the present invention" in the present specification) characteristically comprises the following step:
A) culturing a cell population containing regulatory T cells as primary cultured cells in the presence of a CDK8 and/or CDK19 inhibitor, a TNFR2 agonist, and a TGF-βR agonist or in the presence of a CDK8 and/or CDK19 inhibitor, a TNFR2 agonist, a TGF-βR agonist, and an mTOR inhibitor.

The proliferation method of the present invention can be carried out in the same manner as the production method of the present invention described above, except for use of a cell population containing regulatory T cells as primary cultured cells, and the like, unless otherwise specified.

The cell population containing regulatory T cells used in the proliferation method of the present invention are primary cultured cells isolated from biological tissues, such as bone marrow, umbilical cord blood, and blood. The proportion (number of cells) of regulatory T cells contained in the cell population containing regulatory T cells is, for example, 80% or more, preferably 90% or more, and more preferably 95% or more, and the upper limit is, for example, 100% or less.

The cell population containing regulatory T cells used in the proliferation method of the present invention may be derived from humans, or may be derived from mammals other than humans (non-human mammals), and is preferably derived from humans. Examples of the non-human mammals include mice, rats, hamsters, guinea pigs, rabbits, dogs, cats, pigs, cows, horses, sheep, and monkeys.

In step (A), culture is performed in the presence of (i) a CDK8 and/or CDK19 inhibitor (A), a TNFR2 agonist (M), an mTOR inhibitor (R), and a TGF-βR agonist (T) or in the presence of (ii) a CDK8 and/or CDK19 inhibitor (A), a TNFR2 agonist (M), and a TGF-βR agonist (T). More preferably, culture is performed in the presence of AMT.

The production method of the present invention may further include (2) inducing differentiation of pluripotent stem cells into a cell population containing CD4⁺ T cells before step (1).

The differentiation of pluripotent stem cells into a cell population containing CD4⁺ T cells can be induced according to a known method. Examples of such methods include inducing differentiation of pluripotent stem cells into cells that can differentiate into CD4⁺ T cells, and then inducing differentiation of the cells that can differentiate into CD4⁺ T cells into a cell population containing CD4⁺ T cells. Examples of the cells that can differentiate into CD4⁺ T cells include CD34⁺ cells, mesodermal progenitor cells, CD4CD8 double-negative T cells, CD4CD8 double-positive T cells, and the like, CD34⁺ cells or mesodermal progenitor cells are preferred, hemogenic endothelial cells, hematopoietic stem cells, hematopoietic progenitor cells, progenitor T cells, or mesodermal progenitor cells are more preferred, and hemogenic endothelial cells are particularly preferred. The CD34⁺ cells are (CD34⁺) cells expressing CD34, and particularly are (CD34⁺/CD7⁻) cells expressing CD34 and not expressing CD7. Examples of the CD34⁺ cells include hemogenic endothelial cells, hematopoietic stem cells, and hematopoietic progenitor cells.

The differentiation of pluripotent stem cells into cells that can differentiate into CD4⁺ T cells can be induced according to a known method (see, for example, WO2021/085576 etc.). For example, cells that can differentiate into CD4⁺ T cells can be produced by a feeder-free method, and in particular, "embryonic body method (EB method)" (see AE Grigoriadis et al. (2010). Blood, 115(14): 2769-2776.) in which an embryonic body is formed without using feeder cells to produce hematopoietic progenitor cells is preferred.

Pluripotent stem cells can also be cultured on feeder cells to induce differentiation into hematopoietic stem cells and/or hematopoietic progenitor cells (see WO2011/096482 and WO2013/176197). The feeder cells are preferably stromal cells from the viewpoint of easily inducing differentiation into mesodermal lineage. The stromal cells are preferably OP9 cells, 10T1/2 cells (C3H10T1/2 cells), and the like from the viewpoint of facilitating differentiation into hematopoietic lineage.

The differentiation of pluripotent stem cells into CD34⁺ cells can be performed according to a known method, and when pluripotent stem cells are iPS cells, for example, differentiation of hematopoietic progenitor cells can be induced by the methods described in WO2017/221975, WO2018/135646, and Cell Reports 2 (2012) 1722-1735 to produce CD34⁺ cells.

Examples of the method for inducing differentiation of cells that can differentiate into CD4⁺ T cells into a cell population containing CD4⁺ T cells include an artificial thymic organoid (ATO) method (see WO2017/075389 etc.). The ATO method comprises culturing three-dimensional cell aggregate containing cells that can differentiate into CD4⁺ T cells and stromal cells expressing a Notch ligand, whereby differentiation into CD4⁺ T cells can be induced with high efficiency. The ATO method is a known method, and can be carried out with reference to the description of WO2017/075389, WO2021/085576, and the like. Other methods include the differentiation method (e.g., co-culture with MS5-DLL1/4 stromal cells, OP9 or OP9-DLL1 stromal cells, or EpCAM-CD56⁺ stromal cells) described in WO2021/092581.

When the ATO method is performed, separating cells that can differentiate into CD4⁺ T cells may not be performed before the ATO method is performed, and the ATO method can also be performed on the separated cells that can differentiate into CD4⁺ T cells by a known method (e.g., flow cytometry or magnetic cell separation method).

A nucleic acid for expressing a Notch ligand is preferably introduced into stromal cells. Stromal cell expressing a Notch ligand can be produced by introducing the nucleic acid into the stromal cells. Further, stromal cell expressing a Notch ligand can be produced by introducing the nucleic acid into pluripotent stem cells (particularly iPS cells (especially human iPS cells)) and then differentiating the pluripotent stem cells into stromal cells.

Examples of the stromal cells used in the production method of the present invention include stromal cells induced to differentiate from pluripotent stem cells (particularly iPS cells (especially human iPS cells)), and among them, it is desirable to use cells obtained by inducing pluripotent stem cells (particularly iPS cells (especially human iPS cells)) to differentiate into fibroblasts. The differentiation of pluripotent stem cells into stromal cells can be performed according to a known method, and when pluripotent stem cells are human iPS cells, for example, fibroblasts induced to differentiate from iPS cells can be produced by the method described in PLoS ONE 8(10): e77673, 2013.

The Notch ligand is not particularly limited, and includes canonical Notch ligand and non-canonical Notch ligand described in WO2017/075389. Examples of the canonical Notch ligand include DLL4 (Delta-like ligand 4), DLL1 (Delta-like ligand 1), JAG1 (Jagged 1), JAG2 (Jagged 2), and the like. These can be used singly or in combination of two or more kinds thereof. Examples of the non-canonical Notch ligand include Contactin-1, NOV/CCN3, Contactin-6, Periostin/OSF-2, DLK2/EGFL9, Pref-1/DLK1/FA1, DNER, Thrombospondin-2, MAGP-1/MFAP2, Thrombospondin-3, MAGP-2/MFAP5, Thrombospondin-4, and Netrin-1. Human DLL4 is preferably used as the Notch ligand.

The means for introducing the nucleic acid for expressing the Notch ligand into the stromal cells or pluripotent stem cells is not particularly limited, and various known or general means can be adopted. Typically, the nucleic acid for expressing the Notch ligand is introduced into stromal cells using an expression vector, and is expressed. The expression vector may be linear or cyclic, and may be a non-viral vector such as a plasmid, a viral vector, or a transposon vector.

The means for introducing the expression vector into stromal cells can be made appropriate according to the embodiment. For example, the expression vector can be introduced into stromal cells by a known method, such as a virus infection method, a calcium phosphate method, a lipofection method, a microinjection method, or an electroporation method. The expression vector can be prepared in a form suitable for use in each method by known means, or using commercially available kits as appropriate (according to the instructions thereof).

The expression vector can be introduced into stromal cells by a virus infection method. Examples of viral vectors include retroviral vectors, lentiviral vectors, adenoviral vectors, and adeno-associated viral vectors. When using these viral vectors, a vector containing the nucleic acid for expressing the Notch ligand and a packaging vector (plasmid) of each virus may be transfected into host cells using a corresponding commercially available kit to produce a recombinant virus, and then stromal cells may be infected with the obtained recombinant virus.

In addition to the nucleic acid for expressing the Notch ligand, the expression vector may contain sequences, such as nuclear localization signal (NLS) and multi-cloning site (MCS), if necessary. The expression vector may further contain a nucleic acid (base sequence) encoding "functional genes," such as reporter genes (e.g., genes encoding various color fluorescent proteins), drug selection genes (e.g., kanamycin resistance gene, ampicillin resistance gene, and puromycin resistance gene), and suicide genes (e.g., genes encoding diphtheria A toxin, herpes simplex virus thymidine kinase (HSV-TK), carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella zoster virus thymidine kinase (VZV-TK), xanthine-guanine phosphoribosyltransferase (XGPRT), inducible caspase 9, etc.).

The three-dimensional cell aggregate can be formed, for example, by centrifuging cells that can differentiate into CD4⁺ T cells and stromal cells expressing a Notch ligand. A nucleic acid for expressing a Notch ligand is preferably introduced into stromal cells. The ratio of the stromal cells to the cells that can differentiate into CD4⁺ T cells can be suitably adjusted by a person skilled in the art according to the type of stromal cells and cells that can differentiate into CD4⁺ T cells used, and the like, and is, for example, 100 : 1 to 1 : 100, 90 : 1 to 1 : 90, 80 : 1 to 1 : 80, 70 : 1 to 1 : 70, 60 : 1 to 1 : 60, 50 : 1 to 1 : 50, 40 : 1 to 1 : 40, 30 : 1 to 1 : 30, 20 : 1 to 1 : 20, 10 : 1 to 1 : 10, and the like. For example, when the stromal cells are mouse bone marrow cells (particularly, MS-5), the ratio is preferably 20 : 1 to 1 : 4, and when the stromal cells are pluripotent stem cell-derived stromal cells (particularly, fibroblasts induced to differentiate from iPS cells (particularly human iPS cells)), the ratio is preferably 4 : 1 to 1 : 4 and further preferably 1 : 1.

The medium for culturing the three-dimensional cell aggregate is not particularly limited, and examples thereof include a serum-containing medium, a serum-free medium, or a xeno-free medium, and preferably include a serum-free medium, particularly a serum-free medium containing insulin (further biotin, transferrin, and albumin).

Examples of the basal medium for culturing the three-dimensional cell aggregate include AIM V, X-VIVO-15, NeuroBasal, EGM2, TeSR, BME, BGJb, CMRL 1066, Glasgow's MEM, Improved MEM Zinc Option, IMDM, 199 medium, Eagle's MEM, oMEM, DMEM, Ham, RPMI-1640, Fischer's medium, and the like. These media may be used singly or as a mixture of two or more kinds thereof.

The medium may contain serum or may be serum-free. The medium may further contain serum replacements (e.g., albumin, transferrin, Knockout Serum Replacement (KSR), fatty acid, insulin, collagen precursor, minor element, 2-mercaptoethanol, 3'-thiolglycerol, ITS-supplement, B27 (trademark) supplement, etc.). Serum replacements can be used singly or in combination of two or more kinds thereof.

The medium may further contain one or more substances of lipids, amino acids (non-essential amino acids etc.), L-glutamine, vitamins, growth factors, cytokines, antibiotics, antioxidants, pyruvic acid, buffers, inorganic salts, and the like. As the medium, it is desirable to use a chemically-defined medium that does not contain materials with unknown components, such as serum, because the difference in medium among lots can be reduced, and cells with stable quality can be prepared. In addition, a medium component described in WO2017/075389 and the like can be appropriately blended.

The culture temperature of the three-dimensional cell aggregate is, for example, 20 to 40°C, preferably about 37°C, the CO₂ concentration is, for example, 2% to 10%, preferably 2 to 5%, and the oxygen concentration is, for example, 1 to 20%, preferably 5 to 20%. The cell density at the start of culture can be, for example, about 1.0×10⁴ to 1.0×10¹⁰ cells/mL.

The culture period of the three-dimensional cell aggregate can be suitably adjusted by a person skilled in the art according to, for example, the type of cells for use, such as of stromal cells and cells that can differentiate into CD4⁺ T cells, and is, for example, 4 weeks or more, preferably 5 weeks or more, more preferably 6 weeks or more, and further preferably 7 weeks or more. The upper limit of the culture period is not particularly limited, and is preferably 16 weeks or less, more preferably 14 weeks or less, further preferably 12 weeks or less, and particularly preferably 9 weeks or less.

Since cells other than CD4⁺ T cells may be contained in the cell population containing CD4⁺ T cells obtained by culturing a three-dimensional cell aggregate, CD4⁺ T cells may be isolated and recovered by, for example, flow cytometry (typically, fluorescence activated cell sorting: FACS) using a fluorescently labeled anti-CD4 antibody and a cell sorter with respect to the cell population containing CD4⁺ T cells. Further, CD4SP T cells may be isolated and recovered. Further, effector T cells (Tconv) that are fractionated to be CD25 negative may be isolated and recovered.

When a cell population containing CD8⁺ T cells is used in step (1), step (2) is performed for inducing differentiation of the cell population containing CD8⁺ T cells.

In the production method of the present invention, as the CD4⁺ T cells, cells may be used into which an expression construct (also referred to simply as "CNS-Foxp3" in the present specification) is introduced, the expression construct comprising:
(a) CNS1, CNS2, and CNS3 of Foxp3 gene;
(b) a promoter; and
(c) a nucleic acid encoding FOXP3.

The cells into which the expression construct is introduced are not particularly limited, and may be at any stage of differentiation, and examples thereof include CD4⁺ T cells, pluripotent stem cells, or cells that can differentiate into CD4⁺ T cells. Examples of the method for introducing the expression construct into CD4⁺ T cells, pluripotent stem cells, or cells that can differentiate into CD4⁺ T cells include the methods described above.

The expression construct in the present invention is for expressing FOXP3, and comprises (a) CNS1 (conserved non-coding sequence 1), CNS2 (conserved non-coding sequence 2), and CNS3 (conserved non-coding sequence 3) of Foxp3 gene, (b) a promoter, and (c) a nucleic acid encoding FOXP3. Regulatory T cells can be produced with high efficiency by introducing such an expression construct into CD4⁺ T cells, pluripotent stem cells, or cells that can differentiate into CD4⁺ T cells. The expression construct is not particularly limited as long as it can express FOXP3. The expression construct preferably contains a terminator, a polyadenylation signal, Foxp3 3'UTR, etc., in addition to (a), (b), and (c) mentioned above, and is more preferably one whose components such as these function in cells having the expression construct introduced thereinto.

The base sequences of human Foxp3 gene are registered as RefSeq Accession Nos. NM_001114377 (SEQ ID No. 1) and NM_014009 (SEQ ID No. 2), and the amino acid sequences thereof are also registered as RefSeq Accession Nos. NP_001107849 (SEQ ID No. 3) and NP_054728 (SEQ ID No. 4). These RefSeq IDs are registered on the NCBI website. In the present invention, the above gene also includes degenerate products and variants of genes other than those having the base sequences registered in the database mentioned above, and desirable variants are those encoding proteins having a biological activity equivalent to that of the protein consisting of the above amino acid sequences. Examples of variants include FOXP3 variants having an amino acid sequence that is 80% or more, 85% or more, 90% or more, 91% or more, 92% or more, 93% or more, 94% or more, 95% or more, 96% or more, 97% or more, 98% or more, or 99% or more, identical to the natural amino acid sequence. In the present specification, FOXP3 refers to a protein, and Foxp3 refers to a gene; however, FOXP3 and Foxp3 refer to a gene and protein, respectively, when such an interpretation is appropriate.

The nucleic acid encoding FOXP3 is not particularly limited as long as it is a nucleic acid encoding FOXP3 protein, and preferably cDNA of FOXP3.

CNS1, CNS2, and CNS3 used in the present invention are all derived from Foxp3 gene. CNS1, CNS2, and CNS3 are Foxp3 enhancer elements. The promoter used in the present invention is not particularly limited, and examples include CAG promoter, ubiquitin gene promoter, Foxp3 gene promoter, EF1α promoter, SRα promoter, SV40 promoter, LTR promoter, cytomegalovirus (CMV) promoter, Rous sarcoma virus (RSV) promoter, Moloney murine leukemia virus (MoMuLV) LTR, herpes simplex virus thymidine kinase (HSV-TK) promoter, and the like; and preferably Foxp3 gene promoter. Preferred examples of the base sequences of CNS1, CNS2, CNS3, and promoter of human Foxp3 gene include the base sequences represented by SEQ ID Nos. 5 to 8, respectively. The promoter, CNS1, CNS2, and CNS3 include variants even if they do not have the base sequences described above, and desirable variants are those having a biological activity equivalent to that of the promoter, CNS1, CNS2, and CNS3 consisting of the above base sequences. Examples of variants include those having a base sequence that is 80% or more, 85% or more, 90% or more, 91% or more, 92% or more, 93% or more, 94% or more, 95% or more, 96% or more, 97% or more, 98% or more, or 99% or more, identical to the natural base sequence.

The arrangement (order) of the promoter, CNS1, CNS2, CNS3, and nucleic acid encoding FOXP3 in the expression construct is not particularly limited, and it is preferable that CNS1, CNS2, and CNS3 are located upstream of the promoter; and the order of CNS1, CNS2, CNS3, promoter, and nucleic acid encoding FOXP3 from the 5'-terminal side is more preferred.

The cells into which the expression construct is introduced are not particularly limited as long as they are CD4⁺ T cells, pluripotent stem cells, or cells that can differentiate into CD4⁺ T cells, examples thereof include pluripotent stem cells, hematopoietic stem cells, hemogenic endothelial cells, hematopoietic progenitor cells, progenitor T cells, mesodermal progenitor cells, helper T cells, and the like, pluripotent stem cells are preferred, and iPS cells are more preferred.

Regulatory T cells obtained by the production method and proliferation method of the present invention may be regulatory T cells into which a foreign gene is introduced.

The "foreign gene" is a gene to be introduced from the outside in order to express the desired protein in the regulatory T cells, and can be suitably selected depending on the use of the regulatory T cells.

The foreign gene can be, for example, a gene for expressing a chimeric antigen receptor (CAR), and can further contain a gene for expressing a cytokine and/or a chemokine. As with general or known CARs, the CARs expressed by the regulatory T cells are basically configured such that peptides at sites of (i) an antigen recognition site that recognizes cell surface antigens of cancer cells (e.g., single-chain antibody), (ii) a transmembrane region, and (iii) a signal transduction region that induces the activation of T cells, are linked via a spacer, as needed. The foreign gene can also be, for example, a gene for expressing an exogenous T-cell receptor (TCR). The exogenous TCR means that it is exogenous to T cells into which a nucleic acid encoding the exogenous TCR is to be introduced. The amino acid sequence of the exogenous TCR may be identical to or different from that of the endogenous TCR of the T cells. One or two or more foreign genes may be introduced (e.g., CAR and exogenous TCR).

Examples of the means for introducing the foreign gene into cells include the methods mentioned above. The cells into which the foreign gene is introduced are not particularly limited, and may be at any stage of differentiation, and examples thereof include pluripotent stem cells (particularly, iPS cells), CD4⁺ T cells, regulatory T cells, and the like.

The cell population containing regulatory T cells produced by the production method and proliferation method of the present invention is useful for the treatment of animals (particularly, humans) with an abnormally enhanced immune response, and for example, is useful for the treatment and prevention of immune dysregulation, polyendocrinopathy, enteropathy, X-linked (IPEX) syndrome, graft-versus-host disease (GVHD), rejection in organ transplantation, autoimmune diseases, inflammatory diseases, allergic diseases (hay fever, asthma, atopic dermatitis, eczema, food allergy, food hypersensitivity, urticaria, allergic rhinitis, allergic conjunctivitis, and drug allergy), and the like, but is not limited thereto. The regulatory T cells produced by the production method of the present invention may be used for autologous transplantation or allogeneic transplantation. Further, the regulatory T cells may be used in combination with other drugs.

When performing such cell therapy, from the viewpoint that rejection does not occur, the subject from which cells are isolated for use in the production of regulatory T cells preferably has the same HLA type as a subject to which regulatory T cells are to be administered, and is more preferably the same as the subject to which regulatory T cells are to be administered.

According to the present invention, a medicine comprising a cell population containing regulatory T cells (hereinafter also referred to as "the medicine of the present invention") can be produced. The medicine of the present invention is preferably produced as parenteral preparation by mixing an effective amount of regulatory T cells with a pharmaceutically acceptable carrier according to known means (e.g., the methods described in the Japanese Pharmacopoeia). The medicine of the present invention is preferably produced as a parenteral preparation, such as injection, suspension, or infusion. Examples of parenteral administration methods include intravenous, intraarterial, intramuscular, intraperitoneal, or subcutaneous administration. Examples of the pharmaceutically acceptable carrier include solvents, bases, diluents, excipients, soothing agents, buffers, preservatives, stabilizers, suspensions, isotonic agents, surfactants, solubilizing agents, and the like.

The dose of the medicine of the present invention can be suitably determined depending on various conditions, such as patient's body weight, age, sex, and symptoms, and in general, the medicine of the present invention is administered so that the number of cells per administration for a subject with a body weight of 60 kg is generally 1x10⁶ to 1×10¹⁰ cells, preferably 1×10⁷ to 1×10⁹ cells, and more preferably 5×10⁷ to 5×10⁸ cells. The medicine of the present invention may be administered once or several times. The medicine of the present invention can have a known form suitable for parenteral administration, such as injection or infusion. Further, the medicine of the present invention may contain physiological saline, phosphate buffered saline (PBS), medium, and the like in order to stably maintain the cells. Examples of the medium include RPMI, AIM-V, X-VIVO10, and other media, but are not limited thereto. In addition, pharmaceutically acceptable carriers (e.g., human serum albumin), preservatives, and the like may be added to the medicine for the purpose of stabilization. The medicine of the present invention is applied to mammals, including humans.

As used in the present specification and claims, unless otherwise required by context, singular terms shall include pluralities and plural terms shall include the singular. Thus, singular articles (e.g., "a," "an," "the," and the like in the case of English) should also be understood as encompassing the concepts thereof in the plural form unless specifically noted otherwise.

### Examples

Examples will be given in the following to describe the present invention in more detail. However, the present invention is not limited to these Examples at all.

### Example 1 Differentiation from iPS cells into CD4SP T cells and separation of CD4SP T cells

Step i: A T cell-derived iPS cell strain (4GAD1-4) or a non-T cell-derived iPS cell strain (FFI01s04) was differentiated into a cell population (hereinafter, also referred to as "EB method product") containing hemogenic endothelial cells (HEC) and hematopoietic progenitor cells (HPC) by an embryonic body method (EB method). The present steps i to v are performed in accordance with the method described in WO2021/085576, and differences are extracted and described.

Step ii: In the iPS cell strain 4GAD1-4 derived from T cells, the day on which differentiation into undifferentiated cells was started was day 0, and the EB method product of day 5 was subjected to the ATO differentiation culture method. In the iPS cell strain FFI01s04 derived from non-T cells, the EB method product of day 7 was subjected to the ATO differentiation culture method.

Step iii: In this example, the EB method product recovered without sorting and MS5hDLL4 cells were mixed so that the ratio of the number of cells was the total number of cells contained in the EB method product : MS5hDLL4 = 4 : 1 in 4GAD1-4 and 1 : 20 in FFI01s04, and droplets of the suspension were placed on an insert (Merck Millipore, MILLICELL INSERT 30MM ORGANOTYPIC PTFE 0.4UM 50/PK) and floated on a medium together with the insert to start the ATO differentiation culture method. The medium composition is the same as in the method described in WO2021/085576.

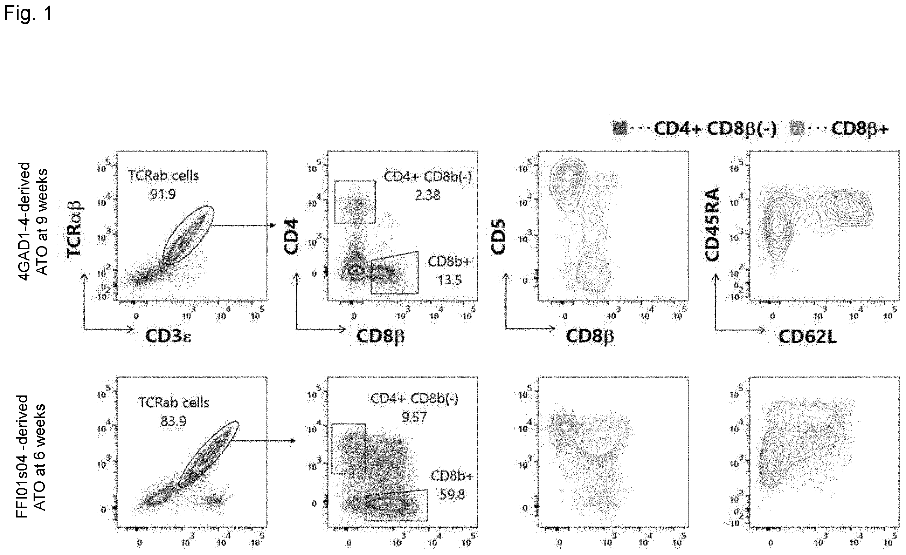

Step iv: When the mixture of the iPS cell-derived EB method product and MS5hDLL4 is cultured for 6 to 9 weeks on the insert floated on this medium, the cells in the mixture were differentiated into CD4SP T cells and CD8SP T cells as shown in Fig. 1.

Step v: ATO of this stage was suspended in 2% FBS/1 mM EDTA/PBS, CD4-positive CD8b-negative cells among CD3-positive TCRab-positive T cells were sorted with a cell sorter, and isolated CD4SP T cells were expanded by culture. For expansion culture, Dynabeads Human T-Activator CD3/CD28 (Thermo Fisher Scientific, hereinafter, referred to as "Dynabeads"), which are beads in which a CD3 antibody and a CD28 antibody are bound, was used. The ratio of beads to cells was 4 : 1 only in the first expansion culture (1 : 1 for the second and subsequent expansion cultures). A medium (hereinafter, referred to as "base medium") was prepared by adding IL-2 (final concentration: 10 ng/mL) and PAA (phosphorylated ascorbic acid) (100 µg/mL) to α-MEM (Thermo Fisher Scientific, MEM-a, nucleosides, and powder were prepared) supplemented with 15% fetal bovine serum (FBS) and ITS (Thermo Fisher Scientific, Insulin-Transferrin-Selenium (ITS-G) (100X)) so as to dilute 100 times as 100 much as PSG (penicillin, streptomycin, and L-glutamine).

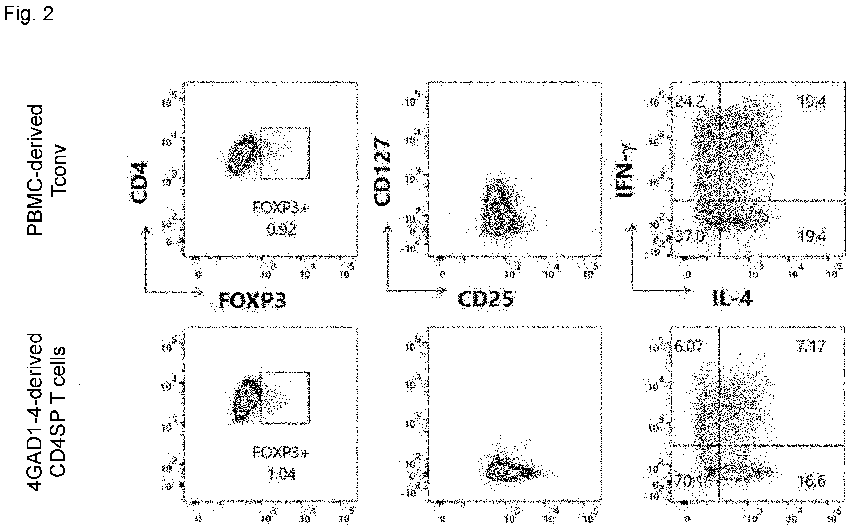

The CD4SP T cells derived from iPS cells showed several hundred to several thousand fold proliferation in initial expansion culture. The cytokine production ability of these CD4SP T cells was evaluated by a method of intracellular staining and flow cytometry. As a result of stimulation with PMA (40 ng/mL) and ionomycin (4 µg/mL) under addition of monensin (2 pM) for 4 hours, as shown in Fig. 2, 13.24% of CD4SP T cells derived from 4GAD1-4 produced interferon-γ (IFN-γ) and 23.77% thereof produced interleukin-4 (IL-4) upon stimulation (both were partially overlapped).

As a control, inflammatory T cells (Tconv) further fractionated in a CD25 negative state among CD4SP T cells were sorted from peripheral blood mononuclear cell fractions (PBMC) of a human living body, cells expanded by culture in a similar manner were placed, and as a result, 43.6% of Tconv also produced interferon-γ (IFN-γ) and 38.8% thereof produced interleukin-4 (IL-4) (similarly, both were partially overlapped). This result showed that the cells obtained by expanding CD4SP T cells obtained from ATO by culture using Dynabeads and IL-2 were FOXP3-negative cells close to Tconv that secretes inflammatory cytokines.

### Example 2 Induction of Treg from CD4SP T cells

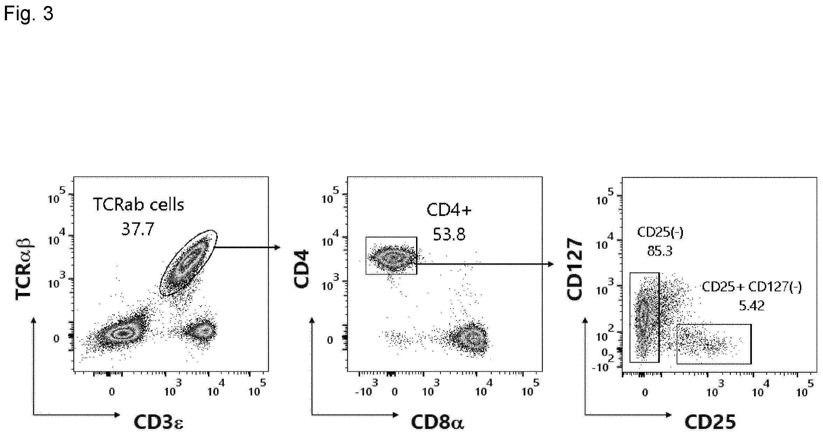

The T cells contained in the peripheral blood mononuclear cell fractions (PBMC) of the human living body are TCRab-positive and CD4-positive CD4SP T cells, and can be further fractionated into the above-described CD25-negative inflammatory T cells (Tconv) and CD25-positive CD127-negative regulatory T cells (Treg) having a function of suppressing inflammation (Fig. 3).

Experiments to maintain or induce FOXP3 were performed using bulk CD4SP T cells, Tconv, or Treg derived from PBMC derived from healthy volunteer donors. To a base medium, 32.52 nM of AS2863619 (MedChemExpress), 100 nM of rapamycin (LKT Labs), 2.5 ug/mL of a TNFR2 antibody (Hycult Biotech, clone MR2-1), and 5 ng/mL of TGF-β (PeproTech, HEK293-derived) were added in the following combination to expand each cell by culture, and the FOXP3 positive rate after 14 days was analyzed by an intracellular staining and flow cytometry.
AR: AS2863619 + rapamycin
ART: AS2863619 + rapamycin + TGF-β
AMT: AS2863619 + TNFR2 antibody + TGF-β
AMRT: AS2863619 + rapamycin + TNFR2 antibody + TGF-β

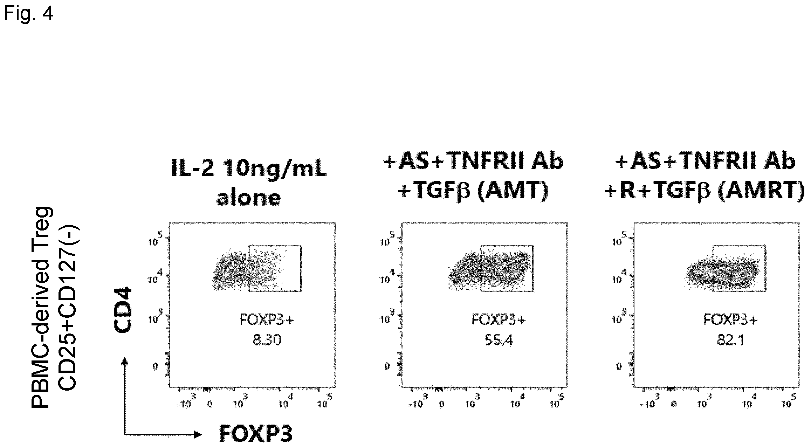

First, Treg was sorted from PBMC, mixed with Dynabeads so that the ratio of beads : cells was 4 : 1, suspended in a base medium, and expanded by culture for 14 days. At that time, for each group of only the base medium, AR, ART, AMT, and AMRT, the expansion ratio and FOXP3 positive rate were analyzed by cell number count and by intracellular staining and flow cytometry. The results are shown in Fig. 4.

FOXP3 was not maintained at a sufficiently high level when culturing with IL-2 alone, and sufficient cell proliferation was not achieved when culturing with AR or ART. On the other hand, FOXP3 expression was highly maintained when culturing with AMT or AMRT.

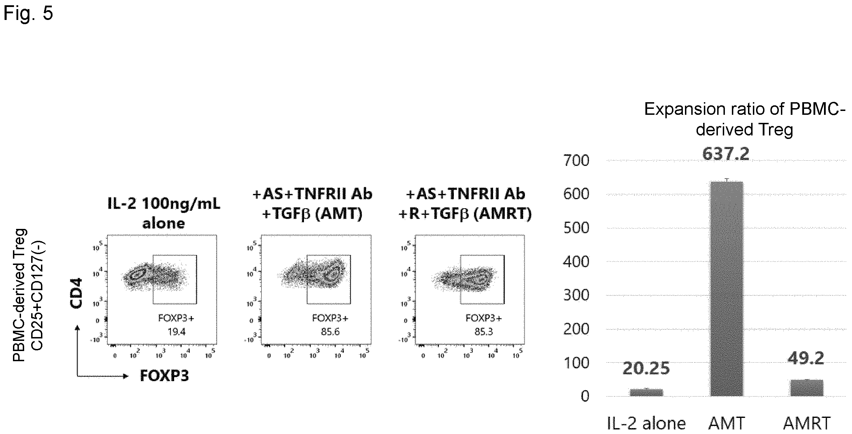

It was found that, when culturing with AMT and 10 ng/mL of IL-2, the FOXP3 positive rate was lower than when culturing with AMRT, but when culturing with AMT and 100 ng/mL of IL-2, an equivalent FOXP3 expression rate was maintained. Therefore, when PBMC-derived Treg is expanded by culture, the IL-2 concentration is then set to 100 ng/mL. The results in this case are shown in Fig. 5. The ratio of FOXP3-positive cells was 85.6% when culturing with AMT and 85.3% when culturing with AMRT. Further, the expansion ratio was increased several times (2.4 times this time) when culturing with AMRT and increased several tens to several hundreds times (31.5 times this time) when culturing with AMT at a higher level as compared to culturing with IL-2 alone.

Next, using CD4SP T cells derived from iPS cell strain 4GAD1-4, FOXP3 induction was performed by each combination described above. Similarly, for each culturing condition group of the base medium only, with AR, with ART, with AMT, and with AMRT, the FOXP3 positive rate was analyzed by the intracellular staining and flow cytometry. At this time, CD4-positive CD8b-negative cells (CD4SP T cells) sorted from ATO and expanded by culture once using a base medium (IL-2 10 ng/mL) were used.

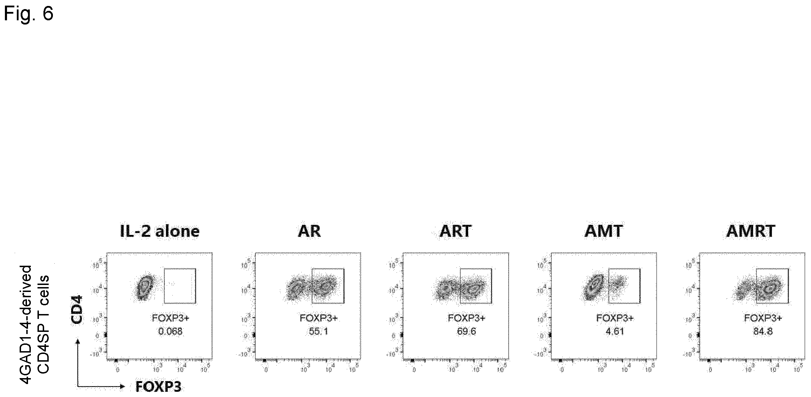

The results are shown in Fig. 6. Culturing with each combination of AR, ART, and AMRT induced high FOXP3 expression with a FOXP3 positive rate of more than 50%. Among them, with AMRT, an extremely high FOXP3 positive rate of 84.8% was observed this time.

### Example 3 Evaluation of Treg

Study on marker expression pattern and inflammatory cytokine secretory capability
It was verified whether the cells in which FOXP3 expression was induced obtained under the conditions described in Example 2 showed CD25-positive CD127-negative CTLA-4-positive expression pattern, which is characteristic of Treg, and whether the cells had a trait of hardly secreting inflammatory cytokines even when stimulated. Analysis was performed by a method of intracellular staining and flow cytometry under conditions stimulated with PMA and ionomycin.

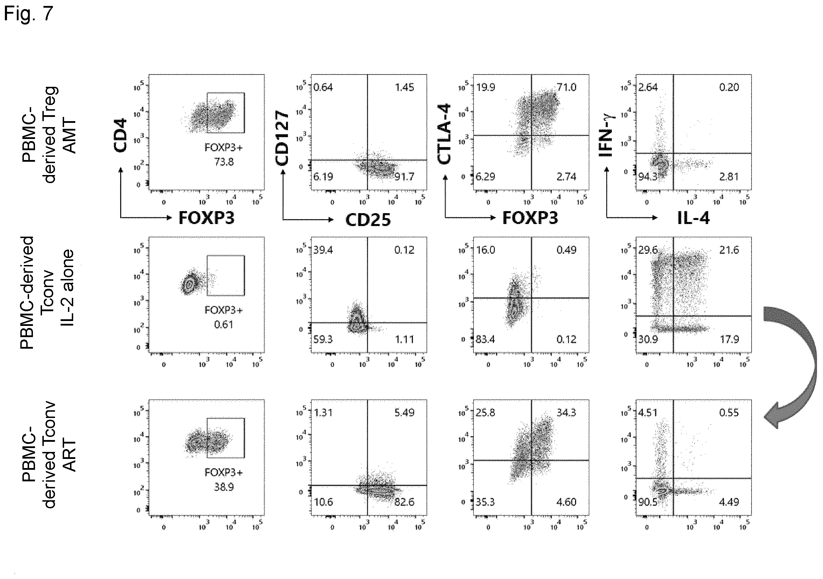

Fig. 7 shows FACS plots of comparing PBMC-derived Tconv expanded by culture under the condition of IL-2 alone (base medium) or the condition of addition of ART to a base medium, using PBMC-derived Treg as a control.

Treg highly expressed FOXP3 and was CD25 positive (this time, positive rate: 93.15%), CD127 negative (positive rate: 2.09%), and CTLA-4 positive (positive rate: 90.9%). The positive rate of IFN-γ was 2.84%, the positive rate of IL-4 was 3.01%, and the Treg hardly secreted inflammatory cytokines.

A change in the expression rate of a marker such as CD25 was observed in PBMC-derived Tconv was expanded by culture between in a base medium as compared with in a base medium added with ART. Specifically, the CD25 positive rate was changed from 1.23% to 88.09%, the CD127 positive rate was changed from 39.52% to 6.80%, and the CTLA-4 positive rate was changed from 16.49% to 60.10%. Further, the positive rate of IFN-γ was decreased from 50.12% to 5.06%, and the positive rate of IL-4 was decreased from 39.5% to 5.04%. From this result, it was confirmed that the addition of ART has an effect of inducing a trait close to Treg other than FOXP3 expression with respect to PBMC-derived Tconv.

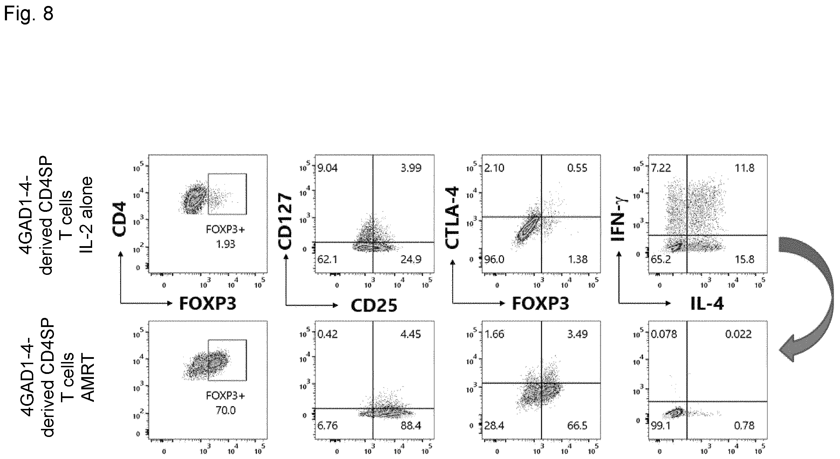

Whether an expression pattern of a marker molecule characterizing Treg other than FOXP3 is observed even when FOXP3 expression is induced in iPS cell-derived CD4SP T cells by AMRT addition was verified by the same method. Fig. 8 shows FACS plots of comparing 4GAD1-4-derived CD4SP T cells expanded by culture under the condition of IL-2 alone (base medium) and the condition of the addition of AMRT to a base medium.

Also in the case of 4GAD1-4-derived CD4SP T cells, by culturing with addition of AMRT to a base medium, CD25 positivity (this time, from 28.89% to 92.85%), a decrease in the expression level of CD127 (from 13.03% to 4.87%), and an increase in the expression level of CTLA-4 (from 2.65% to 5.15%) were observed. Further, the positive rate of IFN-γ was decreased from 19.02% to 0.100%, and the positive rate of IL-4 was decreased from 27.6% to 0.802%.

From this, it was confirmed that the addition of AMRT to a base medium has an effect of inducing a trait close to Treg other than FOXP3 expression in iPS cell-derived CD4SP T cells.

### Study on demethylation of CNS region

A transcription factor binding site that functions to stabilize the expression of the FOXP3 gene called CNS (conserved non-coding DNA sequence) is present in an untranslated region of FOXP3 gene locus (Zheng et al. Nature, 463 (7282), 808-812 (2010)), and is demethylated in Treg. In addition, gene regions specifically demethylated in Treg have been identified, and these are called TSDR (Treg-specific demethylation region).

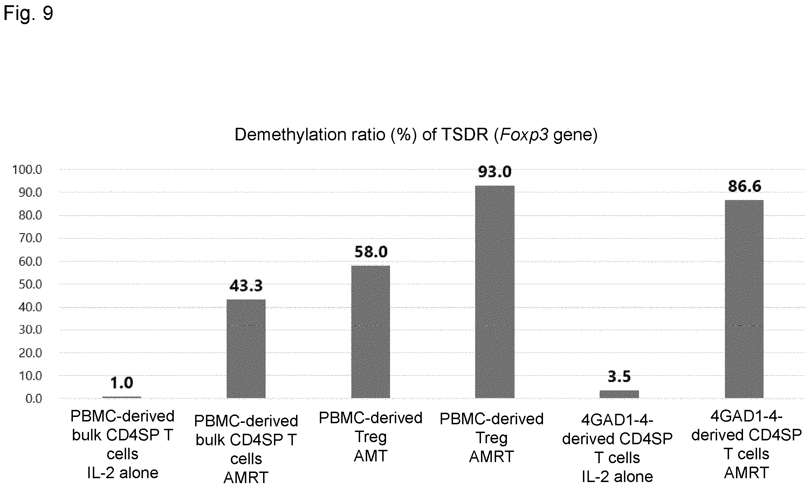

Therefore, whether PBMC-derived bulk CD4SP T cells expanded by culture with a base medium added with AMRT and iPS cell-derived CD4SP T cells expanded by culture with a base medium added with AMRT were associated with demethylation of TSDR (here, FOXP3 CNS2) was analyzed using PureQuant Treg Assay (Thermo Fisher Scientific, the original method is described in Wieczorek et al. Cancer Res., 69(2), 599-608 (2009)). The results are shown in Fig. 9.

In PBMC-derived bulk CD4SP T cells and iPS cell-derived CD4SP T cells expanded by culture with IL-2 alone, TSDR was hardly demethylated. That is, the constitutive expression of FOXP3, which is a transcription factor controlling an immunosuppressive function, is strongly suppressed by methylation. This is also consistent with the fact that these two types of cells show a strong tendency to secrete inflammatory cytokines as shown in Fig. 2.

Furthermore, PBMC-derived bulk CD4SP T cells expanded by culture with a base medium added with AMRT showed a higher demethylation ratio of 43.3% than the case of expansion culture with IL-2 alone, and iPS cell-derived CD4SP T cells expanded by culture with a base medium added with AMRT showed a very high demethylation ratio of 86.6%.

These facts suggest that the expansion culture with a base medium added with the combination of these compounds and cytokine is not a method of transiently expressing FOXP3 by activation, but a method of inducing expression of a stable Treg trait with demethylation of TSDR.

### Example 4 Evaluation of immunosuppressive function

Whether PBMC-derived Treg expanded by culture with AMT and iPS cell-derived CD4SP T cells expanded by culture with AMRT have an immunosuppressive function was verified in vitro and in vivo.

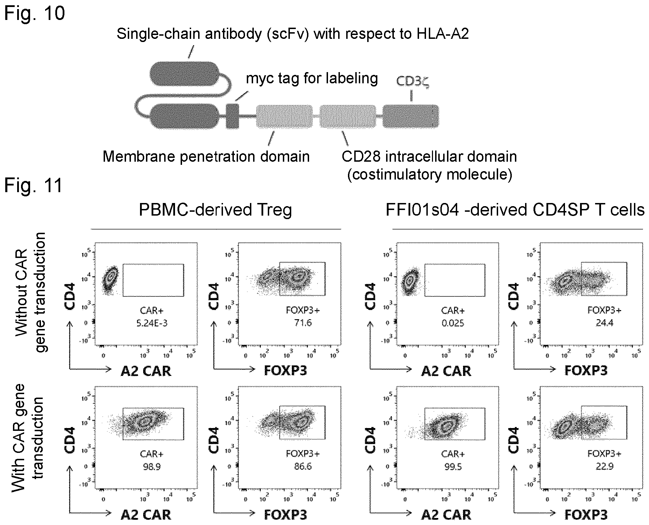

In these experiments, as iPS cells, non-T cell-derived iPS cell strain FFI01s04 not having a reconstituted TCR gene was used. In this case, CD4SP T cells obtained from ATO do not have a specific TCR repertoire. Then, antigen specificity was imparted by gene transduction of a chimeric antigen receptor (CAR). CAR has a chimeric structure in which a single-chain antibody (scFv) in which a light chain (VL) and a heavy chain (HL) of a variable region of a monoclonal antibody to an antigen molecule are bound in series is used as an extracellular domain, and a costimulatory molecule and a CD3z chain are used as an intracellular domain. This time, HLA-A2 was employed as an antigen, and CD28 was employed as a costimulatory molecule (see Fig. 10). By expressing this molecule by gene transduction, T cells (CAR-T cells) expressing this molecule can directly recognize an antigen on the cell surface, here, HLA-A2, in an HLA non-restricted manner.

A gene (SEQ ID NO: 9) of the above-described CAR molecule was transduced into PBMC-derived Treg and iPS cell-derived CD4SP T cells using a retroviral vector. Then, CAR-expressing cells were labeled using a PE-conjugated antibody (Cell Signaling Technology, clone: 9B11) with respect to myc tag, and concentrated by the MACS method using Anti-PE MicroBeads and MS Columns (both available from Miltenyi Biotec).

The CAR expression rate and the FOXP3 expression rate of the cells obtained as a result were analyzed by a flow cytometry. The results are shown in Fig. 11. In the cells subjected to CAR gene transduction, as a result of concentration by MACS, both PBMC-derived Treg expanded by culture with AMT and iPS cell-derived CD4SP T cells expanded by culture with AMRT showed a CAR expression rate close to about 100%. Further, it was also confirmed that FOXP3 expression did not disappear or significantly decrease by CAR gene transduction.

A method for evaluating the function of Treg by transplanting HLA-A2-positive human PBMC into a super immunodeficient strain mouse, and by how much the graft-versus-host disease (GvHD) reaction generated against a heterologous antigen can be suppressed by Treg in which CAR targeting at HLA-A2 is transduced, and as a collateral evidence, how much the same PBMC as that transplanted into a mouse or the proliferation of cytotoxic T cells (CTL) derived therefrom can be suppressed by Treg in vitro has been established as a representative method of functional assay of Treg (MacDonald et al., J. Clin. Invest., 126(4), 1413-1424 (2016), Dawson et al., Sci. Transl. Med. 12, eaaz3866 (2020), Lamarthee et al., Nat. Commun., 12(1), 6446 (2021)).

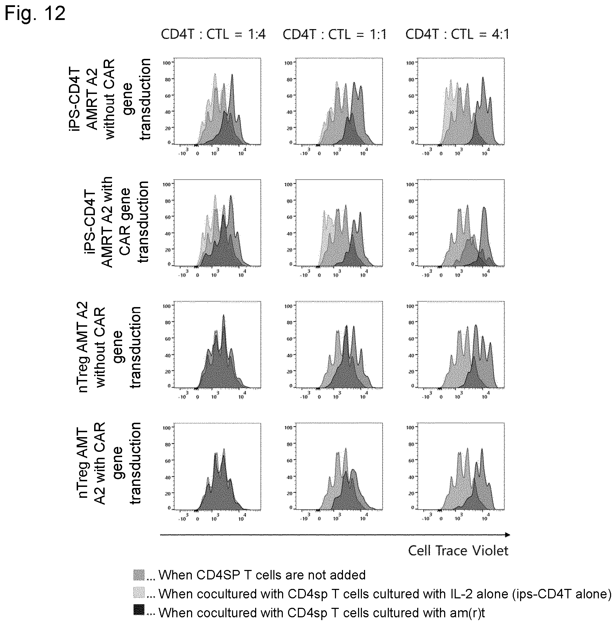

CD8b-positive CTL sorted and expanded by culture from the same PBMC (derived from a donor different from a donor of iPS cells and different from a donor of PBMC with sorted Treg) used in the in vivo experiments described later and PBMC-derived Treg or iPS cell-derived CD4SP T cells (without or with CAR gene transduction for each) were co-cultured. Then, how much CTL proliferation was suppressed was analyzed with a flow cytometry using Cell Trace Violet Cell Proliferation Kit (Thermo Fisher Scientific). CTL is stained with Cell Trace Violet (CTV) dye before co-culture is started, and the extent of proliferation is assessed utilizing the fact that cell division results in daughter cells each having half the fluorescence intensity of their parental cells. The results obtained directly by flow cytometry are shown in Fig. 12.

First, iPS cell-derived CD4SP T cells expanded by culture with IL-2 alone did not show the proliferation inhibitory effect on CTL regardless of the presence or absence of CAR expression. Under the condition in which the number of CD4SP T cells was superior to that of CTLs, such as CD4SP T cells : CTL = 4 : 1, the number of CTLs decreased in the case of using CAR-transduced cells, suggesting that CD4SP T cells exerted a cytocidal effect on CTL. On the other hand, iPS cell-derived CD4SP T cells expanded by culture with a base medium added with AMRT showed an action of suppressing CTL proliferation regardless of the presence or absence of CAR gene transduction.

In order to quantitatively evaluate the cell growth inhibitory action of various cells on CTL, six types of cells, i.e., iPS cell-derived CD4SP T cells expanded by culture with IL-2 alone (without or with CAR gene transduction), iPS cell-derived CD4SP T cells expanded by culture with AMRT (without or with CAR gene transduction), PBMC-derived Treg expanded by culture with AMT (without or with CAR gene transduction) were mixed at each ratio of iPS cell-derived CD4SP T cells (or Treg) : CTL = 1 : 16, 1 : 8, 1 : 4, 1 : 2, 1 : 1, 2 : 1, and 4 : 1, and co-cultured. After co-culture for 96 hours, the fluorescence intensity of CTV was measured with a flow cytometer, and the proportion of divided CTL was calculated. Then, %Suppression = {(Proportion (%) of divided CTL when no CD4SP T cells are added) - (Proportion (%) of divided CTL when CD4SP T cells are added at each ratio)/Proportion (%) of divided CTL when no CD4SP T cells are added} × 100 was further calculated and graphed. The results are shown in Fig. 13. A higher value indicates stronger suppression of CTL proliferation.

All of four types of cells expanded by culture with AMRT or AMT showed an action of suppressing CTL proliferation in a cell number ratio dependent manner. The suppressive action of iPS cell-derived CD4SP T cells did not significantly differ depending on the presence or absence of CAR gene transduction. Also for PBMC-derived Treg, the suppressive action thereof was equivalent regardless of the presence or absence of CAR gene transduction. Further, iPS cell-derived CD4SP T cells showed a stronger CTL growth inhibitory action than PBMC-derived Treg.

From the above, it was confirmed that iPS cell-derived CD4SP T cells expanded by culture with AMRT and PBMC-derived Treg expanded by culture with AMT had a function of suppressing CTL proliferation in vitro.

Subsequently, in vivo experiments were performed using the same HLA-A2-positive PBMC. HLA-A2-positive PBMC and one of four types of iPS cell-derived CD4SP T cells expanded by culture with AMRT (without or with CAR gene transduction) and PBMC-derived Treg expanded by culture with AMT (without or with CAR gene transduction) and were mixed for each 5.0×10⁶, and transplanted into NSG mice by intravenous injection after 2.2 Gy irradiation. Thereafter, the transplantation day was set as day 0, and the body weight was measured every day until day 29 and overall survival was evaluated. The results are shown in Fig. 14.

In the functional assay in vivo, regardless of whether the cells were iPS cell-derived CD4SP T cells or PBMC-derived Treg, transplantation of the cells without CAR gene transduction did not reduce body weight loss and show prolongation of overall survival as compared with transplantation of only PBMC. On the other hand, in both iPS cell-derived CD4SP T cells and PBMC-derived Treg, the cells with CAR gene transduction remarkably decreased body weight loss in mouse and significantly extended overall survival, indicating that GvHD is suppressed. Further, at this time, HLA-A2-positive human PBMC was confirmed in the peripheral blood of the surviving NSG mouse, and it was suggested that the suppression mechanism of GvHD is not due only to cytotoxicity of CAR-positive T cells on HLA-A2-positive human PBMC.

As described above, the immunosuppressive ability as expected in PBMC-derived Treg expanded by culture with AMT was confirmed, and it was confirmed that iPS cell-derived CD4SP T cells expanded by culture with AMRT had a function of suppressing immunity in vivo in the same manner as in PBMC-derived Treg.

The same experiment was also repeated using HLA-A2 positive PBMC derived from another donor. The results are shown in Fig. 15. Here as well, the results showed that iPS cell-derived CD4SP T cells into which CAR gene was transduced decreased body weight loss in mouse and extended overall survival as well as PBMC-derived Treg in which CAR gene was transduced.

### Example 5 Evaluation by iPS cells incorporating CNS-Foxp3

(1) Introduction of construct into iPS cells and Differentiation of construct-introduced iPS cells into HEC

The cell population containing hemogenic endothelial cells used was a floating cell population differentiated from iPS cells (Ff-I01s04 strain: derived from healthy peripheral blood mononuclear cells) provided by the Center for iPS Cell Research and Application, Kyoto University, by known methods (e.g., the methods described in Cell Reports 2(2012)1722-1735 and WO2017/221975).

Specifically, it is as follows. A plasmid sequence was designed and synthesized by introducing a DNA sequence obtained by changing mStrawberry protein sequence in the sequence (MK012431) registered in GenBank to dTomato sequence into a transfer plasmid for producing a third-generation lentiviral vector. This plasmid was transfected into HEK293 lineage cells together with a packaging plasmid and an envelope plasmid for producing a lentiviral vector, and the supernatant containing the produced lentiviral vector was collected, and then concentrated by a high-speed centrifugation to obtain a lentiviral vector carrying CNS-Foxp3 to be introduced into iPS cells. The Ff-I01s04 strain was seeded in a 24-well plate at 1×10⁴ cells/well, protamine was added at a final concentration of 10 pg/mL, and then a lentivirus suspension carrying CNS-Foxp3 gene was directly added to produce virally infected iPS cells. The virally infected Ff-I01s04 strain was seeded at 1×10⁶ cells/well (Day 0) in a ultra-low attachment 6-well plate (Corning), in EB medium (StemPro34 (Gibco) supplemented with 50 ng/ml BMP4 (R&D Systems), 50 ng/ml bFGF (FUJIFILM Wako Pure Chemical Corporation), 50 ng/ml VEGF (R&D Systems), and 2 µM SB431542 (FUJIFILM Wako Pure Chemical Corporation), 10 pg/ml human insulin, 5.5 ug/ml human transferrin, 5 ng/ml sodium selenite (ITS, Gibco), 2 mM L-glutamine (Sigma-Aldrich), 45 mM α-monothioglycerol (Nacalai Tesque, Inc.), and 50 ug/ml ascorbic acid 2-phosphate (Sigma-Aldrich)), and then the cells were cultured under low oxygen conditions (5% O₂) for 4 days (Day 4). Subsequently, the cells were further cultured for 4 days in a medium supplemented with 50 ng/ml bFGF, 50 ng/ml VEGF, and 50 ng/ml SCF (R&D Systems) (Day 8), thereby obtaining a cell population containing HEC.

### (2) Differentiation from HEC into Treg

The cell population containing HEC obtained in (1) was allowed to differentiate into Treg (CD25⁺/FOXP3⁺) with reference to the method described in WO2017/075389 etc.

Specifically, it is as follows. Fibroblasts were induced to differentiate from iPS cells (Ff-I01s04 strain: derived from healthy peripheral blood mononuclear cells) provided by the Center for iPS Cell Research and Application, Kyoto University in order to use them as feeder cells when inducing differentiation of T cells from iPS cells. Subsequently, lentiviruses were used to generate cells that forcibly expressed DLL4 as a Notch ligand under the EF1alpha promoter. More specifically, Ff-I01s04 strain was seeded at 1×10⁶ cells/well in a ultra-low attached 6-well plate (Corning), in a medium prepared by adding Y-27632 (FUJIFILM Wako Pure Chemical Corporation) and CHIR990211 (Tocris Bioscience) to AK03N (Ajinomoto Co., Inc.) (Day 0), and on the next day, the cells were seeded in a 10 cm dish (Corning) coated with 0.1% gelatin (Nacalai Tesque) in Fibroblast medium (alpha-MEM (Invitrogen) supplemented with 20% FBS (Corning), 10 ug/ml human insulin, 5.5 ug/ml human transferrin, 5 ng/ml sodium selenite (ITS, Gibco), 2 mM L-glutamine, 100 U/ml penicillin, 100 ug/ml streptomycin (PSG, Sigma-Aldrich), and 50 ug/ml ascorbic acid 2-phosphate (Sigma-Aldrich)) (Day 1). The medium was changed twice a week, and the culture was continued for 7 days to obtain a cell population containing fibroblasts (Day 8). The obtained iPS cell-derived fibroblasts were seeded at 1×10⁵ cells/well in a 24-well plate coated with gelatin, and on the next day, a lentivirus suspension carrying human DLL4 gene downstream of the EF1alpha promoter and protamine at a final concentration of 10 ug/mL were added, thereby producing iPS cell-derived Fibroblast/DLL4 cells (iFibro/DLL4).

The produced iFibro/DLL4 was used as a support, and co-cultured with the CNS-Foxp3-transduced human iPS cell-derived HEC produced in (1) at a cell ratio of 1 : 1. The medium was RPMI-1640 (FUJIFILM Wako Pure Chemical Corporation) containing 2xB27 supplement (Invitrogen), 1xPSG (Sigma-Aldrich), 1xGlutamax (Invitrogen), 5 ng/mL IL-7 (PeproTech), 5 ng/mL FlT3L (PeproTech), and 50 ug/mL ascorbic acid (Sigma-Aldrich), at a final concentration, respectively, and the cells were co-cultured on 30 mm Millicell (hydrophilic PTFE, pore size: 0.4 µm, height: 5 mm, Merck Millipore) in a 6-well plate (TPP), and cultured for 7 weeks while changing the medium once every three or four days.

### (3) Expansion culture of iPS-Treg

The Treg, differentiated from iPS cells, obtained in (2) were expanded by culture in the following medium containing 10 nM of rapamycin (Merck) and 3 ug/mL of anti-TNFR2 antibody (Hycult Biotech, clone MR2-1) .

Specifically, the CNS-Foxp3-transduced human iPS cell-derived Treg obtained in (2) was cultured in an anti-CD3 antibody (eBioscience)-binding 48-well cell culture plate for 3 days, and then reseeded and cultured in a 24-well G-Rex cell culture plate. The medium was α-MEM (Invitrogen) containing FBS (15%, Corning), L-glutamine-penicillin-streptomycin solution (1/100, Invitrogen, Sigma-Aldrich), insulin-transferrin-selenium supplement (1/100, Invitrogen), ascorbic acid 2-phosphate (50 µg/mL, Sigma-Aldrich), and IL-2 (10 ng/mL, PeproTech), at a final concentration, respectively. For the first three days, anti-CD28 antibody (1.5 µg/mL, BioLegend), anti-CD30 antibody (300 ng/mL, R&D Systems), and a caspase inhibitor (10 pM, R&D Systems) were further added to the above formulation. Culture was carried out for 9 or 10 days while exchanging the medium on the 3rd day after the start of culture and thereafter once every three or four days. When the cells obtained after the first expansion culture were recovered, CD4-positive fraction was fractionated using TregCD4 Microbeads (Myltenyi Biotec). The expansion culture was further continuously performed twice, and the expansion culture was performed three times in total.

### (4) Expansion culture of iPS-Treg with AMRT

The iPS-Treg obtained in (3) was further expanded by culture in a medium further added with AS2863619 as a CDK8/19 inhibitor and TGF-β (AMRT) .

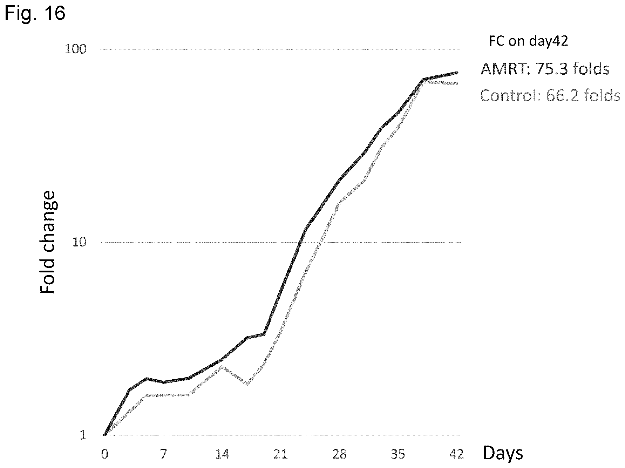

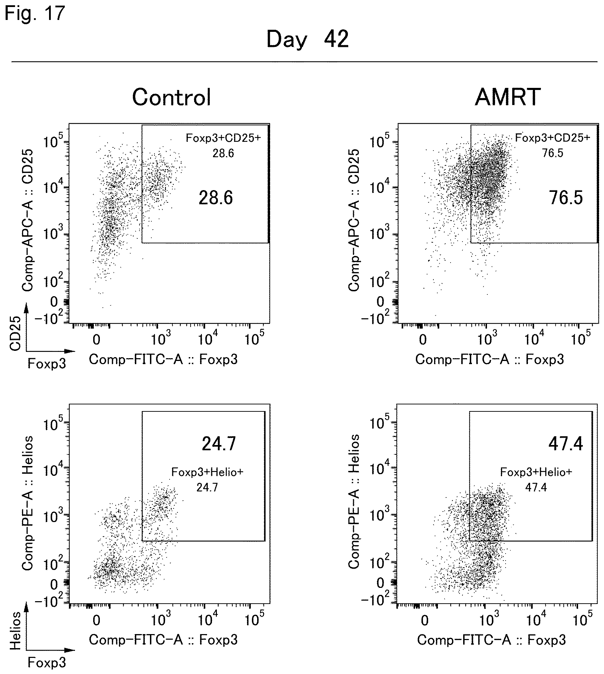

Specifically, the iPS-Treg obtained in (3) was further expanded by culture in the same conditions (Control) as in (3) or in a medium further added with AS2863619 (MedChemExpress, 30 nM) and TGF-β (Peprotech, 5 ng/mL) (AMRT). The culture period was 14 days, and expansion culture was repeated three times. The cell proliferation results at this time are shown in Fig. 16. Each cell population on the final day of expansion culture was stained with the following antibodies (Zombie NIR, BV510 CD3, BV421 CD4, PE/Cy7 CD8β, APC CD25, FITC FOXP3, and PE Helios). The results are shown in Fig. 17. Fig. 17 shows Zombie NIR⁻CD3⁺CD4⁺CD8β⁻ cells.

In the group expanded by culture under the AMRT conditions, no significant difference was observed in cell proliferation as compared with the control group, but CD25⁺FOXP3⁺ fraction and FOXP3⁺Helios⁺ fraction were obtained in an amount of 76.5% and 47.4%, respectively, which were higher than those in the control group (28.6% and 24.7% in the control group, respectively).

### Example 6 Evaluation of suppressive function of CNS-Foxp3-transduced iPS cell-derived Treg

Using Treg expanded by culture in Example 5, analysis by flow cytometry after co-culture with human PBMC-derived allogeneic T cells was performed.

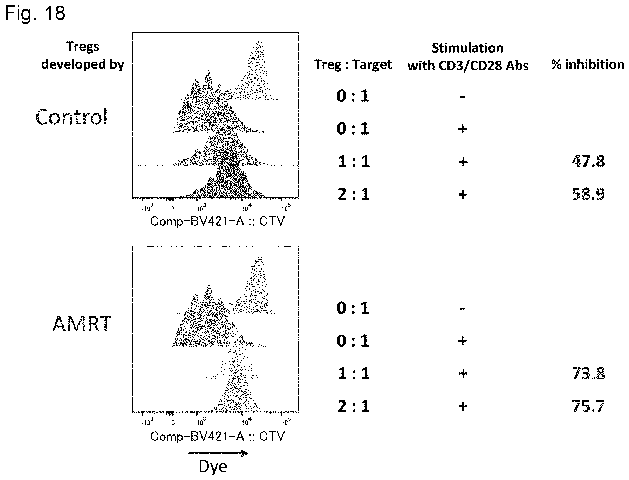

Specifically, PBMC, derived from a donor different from that of Treg, was treated with or without an anti-CD3 antibody and stained with Cell Trace Violet (Thermo Fisher Scientific) to use as target cells, and cocultured with Tregs at a cell number ratio of Treg (control group in Example 5 or group expanded by culture under AMRT conditions) : PBMC = 0 : 1, 1 : 1, and 2:1. The medium was α-MEM (Invitrogen) containing, FBS (15%, Corning), L-glutamine-penicillin-streptomycin solution (1/100, Invitrogen, Sigma-Aldrich), insulin-transferrin-selenium supplement (1/100, Invitrogen), and ascorbic acid 2-phosphate (50 µg/mL, Sigma-Aldrich), at a final concentration, respectively, and the cells were cultured for 5 days. Each cell population was stained with the following antibodies (Zombie NIR, PE/Cy7 CD3, and PE HLA-A24). The results are shown in Fig. 18.

In the group co-cultured with iPS-Treg, cell division of target cells was suppressed in a Treg cell number dependent manner. Further, regarding the suppressive function, a stronger effect was observed in Treg cultured under AMRT conditions than in the control group. In the case of Treg : Target = 2 : 1, the suppression rate of the control group was 58.9%, whereas the suppression rate of 75.7% was observed in the cell population cultured under the AMRT condition.

### Example 7 Candidate gene for inducing differentiation of CD4SP T cells

Human induced pluripotent stem cell strains

Human iPS cell strain TKT3V1-7 was derived from antigennonspecific CD3 T cells of healthy volunteers using a retroviral vector carrying OCT3/4, KLF-4, SOX-2, and C-MYC (Molecular Therapy 29 10, 2013 kaneko S).

### Cell strains

To generate MS5-hDLL4, MS5 cells (K Itoh et al., 1989. Exp Hematol 17, 145-153) were transformed with a retroviral vector encoding full-length human DLL4. Up to 5% of DLL4-expressing cells were sorted by FACS using an anti-DLL4 antibody and passaged in DMEM/10% fetal bovine serum (FCS). Stable expression of DLL4 was confirmed by flow cytometry after several weeks of culture.

### Production of iHPCs from iPSC

The method of differentiating iPSC (iPS cells) into T cells was previously reported (Nishimura et al., 2013, Cell Stem Cell 12, 114-126). iPSC was expanded by culture on iMatrix-511 of StemFit AK02N for 6 to 7 days and dissociated into single cells with 0.5x TryPLE select (Thermo Fisher Scientific) . The 3 to 6×10⁵ of all cells were resuspended in StemFit AK02N supplemented with 10 µM Y-27632 (FUJIFILM Wako Pure Chemical Corporation) and 10 µM CHIR99021 (Tocirs Bioscience), and cultured for 24 hours on a 6-well ultra-low adhesion plate (Corning). Thereafter, EB was recovered and precipitated on the bottom of the tube, and resuspended in a 2 mL/well of "EB basal medium" (StemPro-34 (Thermo Fisher Scientific) supplemented with 10 ng/mL penicillin/streptomycin (Sigma-Aldrich), 2 mM Glutamax (Thermo Fisher Scientific), 50 ug/mL ascorbic acid-2-phosphate (Sigma-Aldrich), 4×10⁻⁴ M monothioglycerol (MTG, Nacalai Tesque) and 1x insulin-transferrin-selenium solution (ITS-G, Thermo Fisher Scientific)) further added with 50 ng/ml recombinant human (rh)BMP-4 (R&D Systems), 50 ng/ml rhVEGF (R&D Systems), and 50 ng/ml bFGF (FUJIFILM Wako Pure Chemical Corporation). After 24 hours, 6 µM SB431542 (FUJIFILM Wako Pure Chemical Corporation) was added. After 4 days, the differentiated EB was collected, washed, and then resuspended in a 2 mL/well of EB basal medium supplemented with 50 ng/mL rhVEGF, 50 ng/mL rhbFGF, and 50 ng/mL rhSCF (R&D Systems), and cultured for 2 days. After 7 days, the differentiated EB was collected again, washed, and resuspended a 2 mL/well of EB basal medium supplemented with 50 ng/mL rhVEGF, 50 ng/mL rhbFGF, 50 ng/mL rhSCF, 30 ng/mL rhTPO (PeproTech), and 10 ng/mL FLT3L (PeproTech). From day 7, cells were harvested every 2 to 3 days and replaced with a fresh medium. The culture was maintained in 5% CO₂/5% O₂/90% N₂ environment for the first 7 days, and maintained in 5% CO₂ environment thereafter. The differentiated iHPCs was filtered through a 40 µm cell strainer and cryopreserved at -80°C using TC-protector (Bio-Rad antibodies).

### 2D feeder-free differentiation of iHPC into progenitor T cells

Previously reported iHPC derived from TKT3v1-7 was used (Iriguchi et al, 2021, Nat Commun 12, 430.10.1038/s41467-020-20658-3). T cell differentiation was induced on a rhDL4-coated plate prepared the day before iHPC seeding. A rhDL4/Fc chimeric protein solution (5 µg/mL, Sino Biological) was diluted with an equal amount of retronectin (5 µg/mL, Takara Bio Inc.) and added at 150 µL/well in a 48-well plate, and plates were incubated overnight at 4°C. The coating solution was removed immediately before adding a T cell differentiation medium.

For seeding iHPC, 11 to 14 EBs were collected and dissociated into single cells by TryPLE Select (Thermo Fisher Scientific) treatment. 2000 CD34⁺/CD43⁺ cells were directly FACS-sorted into wells of a DL4-coated plate containing a T cell differentiation medium consisting of αMEM (Thermo Fisher Scientific) supplemented with 15% FBS (Corning), 100xITS-G (1x), 55 µM 2-mercaptoethanol (Thermo Fisher Scientific), 50 ug/mL ascorbic acid-2-phosphate, 2 mM Glutamax, 50 ng/mL rhSCF, 50 ng/mL rhTPO, 50 ng/mL rhIL-7, 50 ng/mL FLT3L, 30 nM rhSDF-1α (PeproTech), and 15 µM SB203580 (Tocris Bioscience). Most of the medium (80%) was changed every other day. The differentiated cells were transferred to a new DL4-coated plate on day 7 and 1 to 2×10⁵ cells/well were transferred to a new DL4-coated plate on day 14. The culture was maintained in 5% CO₂ environment.

### Maturation of progenitor T cells into CD8 single-positive T cells

DL4 cells on day 21 were stimulated with 500 ng/mL anti-CD3 monoclonal antibody (clone: OKT3, eBioscience) in a maturation medium consisting of αMEM, 15% FBS, 100xITS-G (1x), 50 ug/mL ascorbic acid-2-phosphate, 100xPSG (1x, Sigma-Aldrich), 10 ng/mL rhIL-7, 10 ng/mL rhIL-2 (PeproTech), and 10 nM dexamethasone (Fuji Pharma Co., Ltd.). After 3 days, the cells were recovered, washed, resuspended in an OKT3-free maturation medium, and cultured for 4 days in an environment containing 5% CO₂ at 37°C.

### Maturation of iHPC into CD4 and CD8 single-positive T cells

The previously reported method was used with some modifications (Seet et al., 2017, Nat Methods 14, 521-530.10.1038/nmeth. 4237). Briefly, 1×10⁶ MS5-hDLL4 cells and 1×10⁵ purified iHPC were loaded in a 1.5 mL microcentrifuge tube to prepare a dense MS5-hDLL4-iHPC mixture. The mixture was adjusted to 15 µL per well. For each culture, a 0.4 µm Millicell Transwell insert (EMD Millipore, Billerica, MA; Cat. PICM0RG50) was placed in a 6-well plate containing 1.5 mL of organoid culture per well. The medium was completely replaced every 3 to 4 days.

### Single-cell RNA-seq

The isolated single cell suspension was subjected to dropletbased massively parallel scRNA-seq using Chromium Single Cell 50 Reagent Kit (10x Genomics) according to manufacturer's instructions. Briefly, the cell suspension was loaded at 700 to 1,200 cells/µL for the purpose of capturing 3,000 cells per well. GEM droplets were generated on 10x Chromium Controller, each cell was labeled with a specific barcode, and each transcript was labeled with a unique molecular identifier (UMI) upon reverse transcription. Barcoded cDNA was isolated with Dynabeads MyOne SILANE bead cleanup mixture. cDNA was amplified by PCR and purified by SPRI bead cleanup.

For the gene expression library, 50 ng of amplified cDNA was used, and library preparation consisting of fragmentation, end repair, A-tailing, adaptor ligation, and sample index PCR was performed according to manufacturer's instructions. The library was sequenced with a NovaSeq sequencer (Illumina, Inc.).

### Single-cell RNA-seq data processing

Sequence reads of organoid culture samples were aligned to human GRCh38 genomic references and tag counts from genes and antibodies were quantified as UMIs using Cell Ranger v3.1.0 software (10x Genomics). The UMI count matrix was imported into R v3.6.1 software Seurat v3.1.1 package and further data processing was performed using Seurat package. Cells having a proportion of the number of mitochondrial genes of 20% or more were excluded from further analysis as dead cells or damaged cells. The gene number and the tag count derived from the antibody were normalized by a global scale normalization method and a centered logarithmic ratio normalization method, respectively. Intracellular, differentially expressed genes were selected by "vst" method of Seurat package, and principal component analysis was performed. The calculated principal components were used for dimension reduction and graph-based clustering.

BAM files (GSE148978) of human thymocyte samples were downloaded from the SRA repository. The BAM file was converted into a FASTQ sequence file using bamtofastq software v1.3.2 (10x Genomics). Sequence reads were aligned to human GRCh38 genomic references and gene tag counts were quantified as UMIs using Cell Ranger v5.0.1 software. Cells with the number of UMIs of less than 200 or 10000 or more, or cells with a proportion of the number of mitochondrial genes of 5% or more were excluded from further analysis. Human thymocyte datasets were integrated by Seurat's standard integration method and data processing was performed. Clusters composed of cells with the same number of UMIs or cells derived from one donor were deleted.

Sequence reads of 2D culture samples were aligned to human GRCh38 genomic references and gene tag counts were quantified as UMIs using Cell Ranger software v5.0.1. Data processing was performed with Seurat v3.2.3. package.

Datasets of organoid culture, two-dimensional culture, and human thymocyte samples were integrated by Seurat's standard integration method and data processing was performed.

Genes with different expression levels in the cluster relative to other clusters were defined as those having a fold change of 2 or more and a p-value of less than 0.05 by Wilcoxon rank sum test.

### Pseudotime analysis

The integrated dataset was converted to the monocle3 v0.2.3.0 package (Cao et al., 2019, Nature 566, 496-502.10.1038/s41586-019-0969-x; Qiu et al., 2017, Nat Methods 14, 309-315.10.1038/nmeth.4150; Qiu et al., 2017, Nat Methods 14, 979-982.10.1038/nmeth. 4402; Trapnell et al., 2014, Nat Biotechnol 32, 381-386.10.1038/nbt. 2859) format and data processing was performed. The Seurat embedding space of UMAP was used for further analysis. The embedding space of the main UMAP was fitted to obtain a trajectory graph. The node of the DN cells was taken as a start, and the pseudo time was determined. According to Moran's I test, genes with different expression levels were extracted in paths with different trajectories, and the identified genes with different expression levels were classified into gene set modules of co-regulated genes together with trajectories and pseudo times.

### Gene candidates involved in differentiation transition to CD4SP T cells in organoid culture were identified by pseudotime analysis

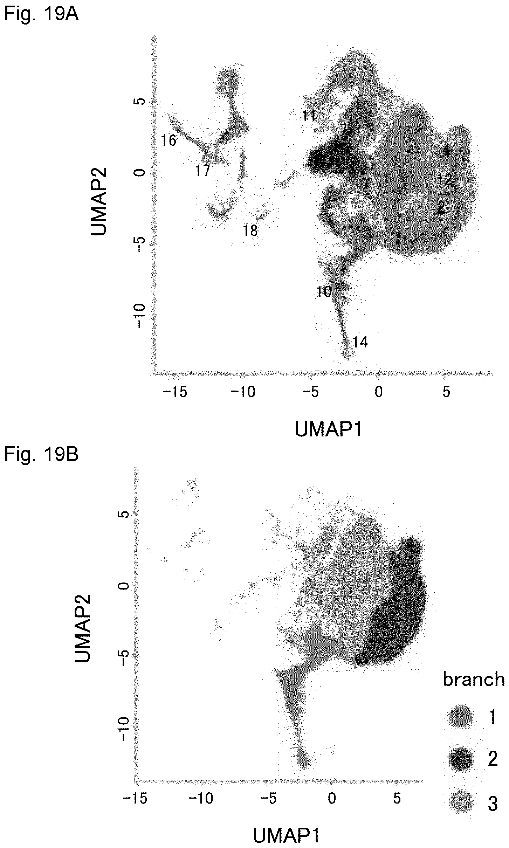

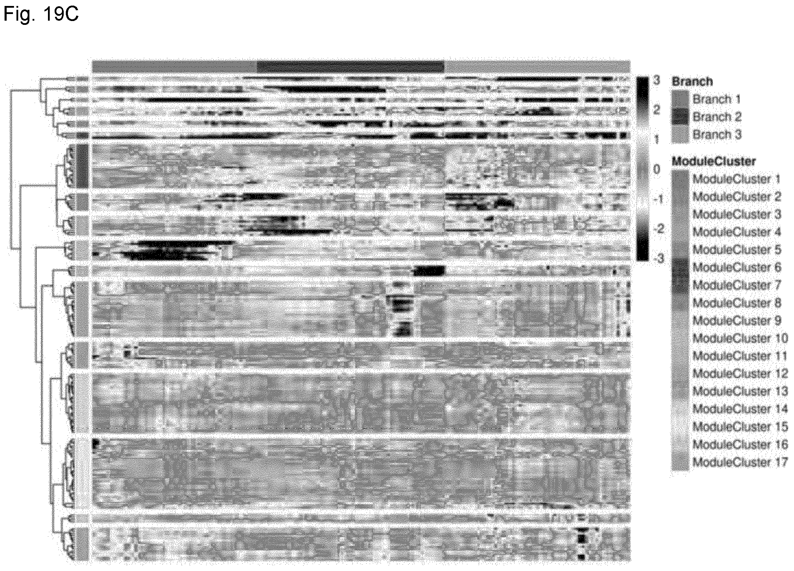

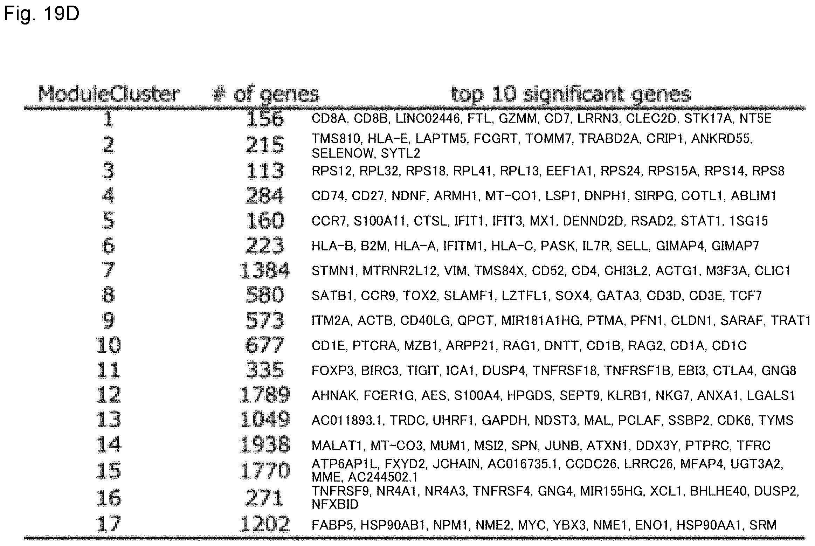

To identify key genes in CD4SP T-iPS-T differentiation from DP (CD4⁺CD8⁺ double positive) cells, pseudotime analysis was performed on an integrated single cell RNA-seq dataset of all cells in organoid culture and thymocytes (Fig. 19A). Clusters specific to organoid culture were excluded from the integrated dataset. As a result of trajectory analysis, the cell dataset was divided into three branches (Fig. 19B). These branches divided the cells into immature cells (red), CD4SP line (green), and CD8SP line (blue) (Fig. 19B). Significantly controlled genes were further subjected to hierarchical clustering analysis, yielding 17 modules (Fig. 19C; the top 10 genes of each module cluster are shown in Fig. 19D). In order to examine genes with different expression in the process of T cell differentiation from DP to CD4SP, attention was paid to module clusters #7, #8, and #9 containing genes with increased expression in the process of differentiation from immature stage to CD4SP, and module clusters #2, #5, and #6 containing genes with reduced expression in the relatively early stage of differentiation to CD8SP. The genes included in these module clusters were assumed to be candidate genes for the transition from DP phase to CD4SP T cells.

### Sensitized helper population is included in 2D differentiated samples

To identify potential genes/pathways of CD4SP T cell differentiation by feeder-free culture, the single cell transcriptomes of cells obtained from 2D culture (CD8SP only) and organoid culture (CD4SP and CD8SP cells) were compared.

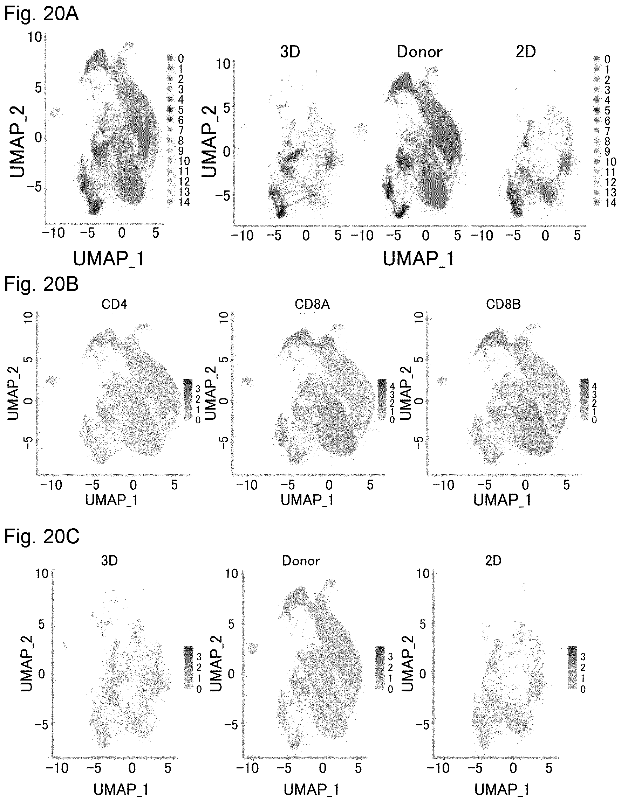

Datasets of 6-week organoid culture, 2D culture, and thymocytes were integrated and processed as UMAP plots (Fig. 20A, left). Nonintegrated datasets also showed a common cluster (Fig. 20A, right). Thymocytes showed various clusters. Clusters #1 and #3 showed overwhelmingly increased CD8 expression, whereas Clusters #0 and #2 showed CD4 expression. These cluster gene expression was higher in the thymocyte dataset than in the culture data. 2D and organoid culture clusters showed T cell-related gene expression. Unexpectedly, some cells from 2D culture samples also showed in the cluster (Fig. 20C) CD4 expression in thymocytes and organoid culture data (Figs. 20B and 20C). Further, SELL associated with Tscm (Cheng-Chi Chao et al., 1997, J Immunol 159, 1686-1694) was expressed in cluster #0, and BACH2 associated with T cells (Rahul Roychoudhuri et al., 2013, Nature 498, 506-512.10.1038/nature12199) was expressed in cluster #2. These results indicate that some cells in 2D culture have helper functions.

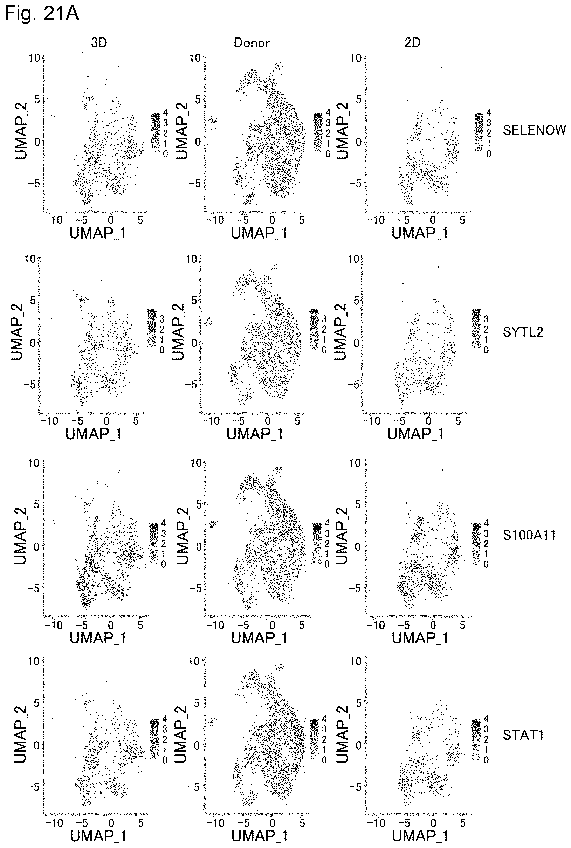

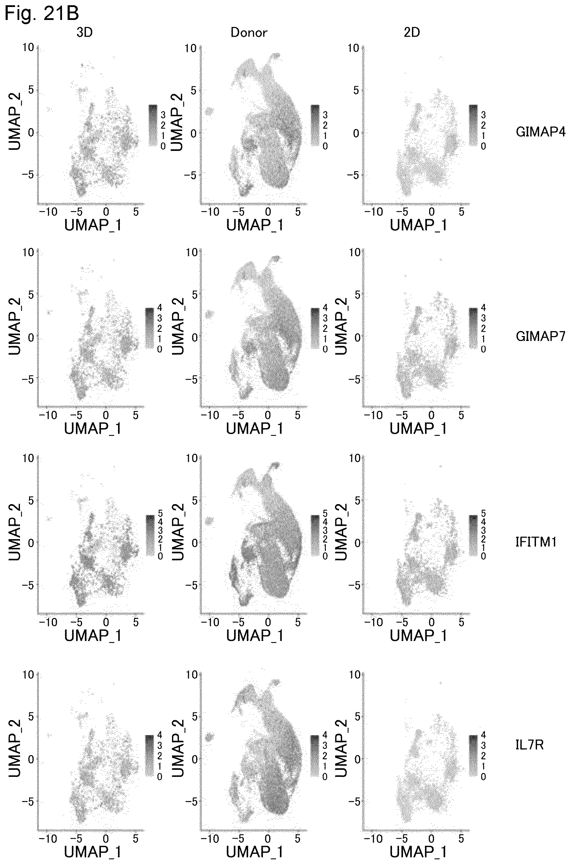

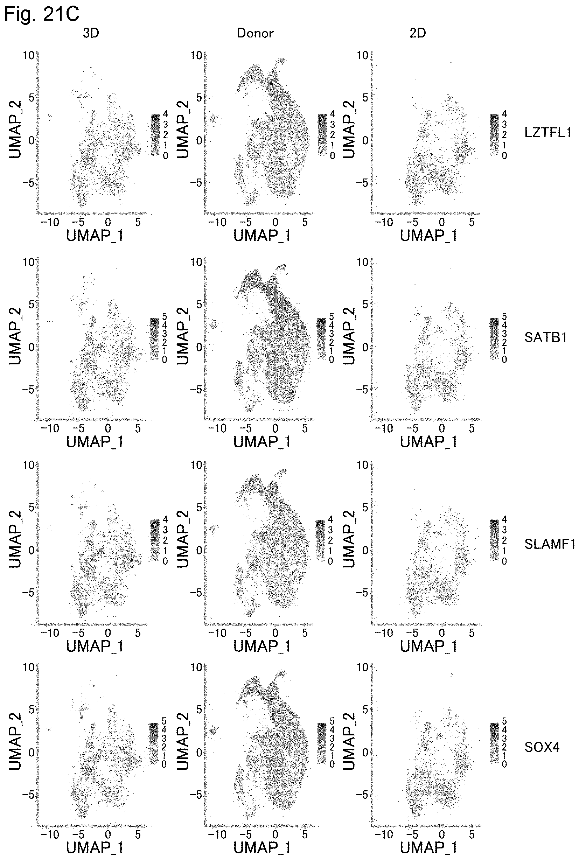

In order to elucidate the mechanism of low expression of CD4 in 2D culture, gene expression kinetics in the process of transition to mature CD4SP T cells were compared. Utilizing the controlled gene cluster identified in the pseudotime analysis (Fig. 19D), and assuming that the expression differences were related to CD4 expression in the organoid culture, the expression of the top 10 genes of module clusters #2, #5, #6, #7, #8, and #9 of the 2D culture dataset was evaluated. For potential helper functional clusters (Fig. 20C), SELENOW (ENSG00000178980) and SYTL2 (ENSG00000137501) of module cluster #2, S100A11 (ENSG00000163191) and STAT1 (ENSG00000115415) of module cluster #5, GIMAP4 (ENSG00000133574), GIMAP7 (ENSG00000179144), IFITM1 (ENSG00000185885), and IL7R (ENSG00000168685) of module cluster #6, and LZTFL1 (ENSG00000163818), SATB1 (ENSG00000182568), SLAMF1 (ENSG00000117090), and SOX4 (ENSG00000124766) of module cluster #8 showed a higher expression level in the organoid culture than in 2D culture (Ensemble gene ID is shown in parentheses) (Fig. 21). It is considered that differentiation from pluripotent stem cells (particularly, iPS cells) etc. into desired T cells (particularly, CD4SP T cells) can be induced by controlling these genes.

This application is based on Japanese Patent Application No. 2022-47437 filed on March 23, 2022 and Japanese Patent Application No. 2022-152604 filed on September 26, 2022 in Japan, the contents of which are encompassed in full herein.

## Claims

1. A method for producing a cell population containing regulatory T cells, the method comprising
(1) culturing a cell population containing pluripotent stem cell-derived CD4⁺ T cells in the presence of at least one substance selected from the group consisting of a CDK8 and/or CDK19 inhibitor, a TNFR2 agonist, an mTOR inhibitor, and a TGF-βR agonist.

2. The method according to claim 1, wherein step (1) is performed in the presence of a CDK8 and/or CDK19 inhibitor and an mTOR inhibitor.

3. The method according to claim 1, wherein step (1) is performed in the presence of a CDK8 and/or CDK19 inhibitor, an mTOR inhibitor, and a TGF-βR agonist.

4. The method according to claim 1, wherein step (1) is performed in the presence of a CDK8 and/or CDK19 inhibitor, a TNFR2 agonist, an mTOR inhibitor, and a TGF-βR agonist.

5. The method according to claim 1, wherein the CDK8 and/or CDK19 inhibitor is at least one selected from the group consisting of 4-[1-(2-methyl-1H-benzimidazol-5-yl)-1H-imidazo[4,5-c]pyridin-2-yl]-1,2,5-oxadiazol-3-amine, 3-{1-[1-(4-methoxyphenyl)piperidin-4-yl]-4-methyl-1H-imidazo[4,5-c]pyridin-2-yl}pyrazin-2-amine, and siRNA of CDK8 and/or CDK19.

6. The method according to claim 1, wherein the mTOR inhibitor is rapamycin.

7. The method according to claim 1, wherein the TNFR2 agonist is a TNFR2 agonist antibody.

8. The method according to claim 1, wherein the TGF-βR agonist is TGF-β.

9. The method according to claim 1, wherein the regulatory T cells are CD25⁺/FOXP3⁺ cells.

10. The method according to claim 1, wherein the CD4⁺ T cells are CD4⁺/CD8⁻ T cells.

11. The method according to claim 1, further comprising (2) inducing differentiation of pluripotent stem cells into a cell population containing CD4⁺ T cells before step (1) .

12. The method according to claim 1, wherein the pluripotent stem cells are iPS cells.

13. The method according to claim 11, wherein a three-dimensional cell aggregate is cultured in step (2), the three-dimensional cell aggregate containing cells that can differentiate into the pluripotent stem cell-derived CD4⁺ T cells and stromal cells expressing a Notch ligand.

14. A cell population comprising regulatory T cells obtained by the method according to claim 1.

15. A medicine comprising the cell population containing regulatory T cells according to claim 14.

16. The medicine according to claim 15, for use in prevention and/or treatment of autoimmune disease, graft-versus-host disease, or transplant rejection.

17. A method for preventing and/or treating autoimmune disease, graft-versus-host disease, or transplant rejection, the method comprising administering the cell population containing regulatory T cells according to claim 14 to a subject in need thereof.

18. The cell population containing regulatory T cells according to claim 14, for use in prevention and/or treatment of autoimmune disease, graft-versus-host disease, or transplant rejection.

19. Use of the cell population containing regulatory T cells according to claim 14 in the production of a medicine for use in prevention and/or treatment of autoimmune disease, graft-versus-host disease, or transplant rejection.

20. The method according to claim 1, wherein the CD4⁺ T cells are cells into which an expression construct is introduced, the expression construct comprising:
(1) (a) conserved non-coding sequence (CNS) 1, CNS2, and CNS3 of Foxp3 gene;
(b) a promoter; and
(c) a nucleic acid encoding FOXP3.
